# EUROPEAN PATENT APPLICATION

(11) **EP 4 541 908 A1**
(43) Date of publication of application: **23.04.2025**
(21) Application number: 23824230.9
(22) Date of filing: 14.06.2023
(51) Int. Cl.: C12Q 1/6883, A61K 48/00, A61K 31/7088, A61P 19/00

(54) **COMPOSITION FOR PREVENTION OR TREATMENT OF OSTEOARTHRITIS AND USE THEREOF**

(30) Priority: 15.06.2022 KR 20220073019
(71) Applicant: INDUSTRY-ACADEMIC COOPERATION FOUNDATION, YONSEI UNIVERSITY, Seoul 03722 (KR)
(72) Inventor: KIM, Sung-Hwan, Seoul 06216 (KR); LEE, Jin Woo, Seoul 06588 (KR); YOON, Dong Suk, Goyang-si Gyeonggi-do 10551 (KR)
(74) Representative: Müller, Christian Stefan Gerd
(86) International application number: PCT/KR2023/008204
(87) International publication number: WO 2023/244016

(57) **Abstract**

The present invention relates to novel long non-coding RNAs (lncRNA) involved in the regulation of hypertrophy of chondrocytes, wherein RUNX2 was regulated at the post-translational level during hypertrophic differentiation of the chondrocyte cell line TC28a2 and two novel RUNX2-binding lncRNAs among numerous lncRNAs were identified, leading to the present invention. It is expected that bone-related diseases can be diagnosed by measuring the expression of the identified specific lncRNAs, and furthermore, the vascularization or calcification of articular cartilage can be effectively suppressed by inhibiting the hypertrophy of chondrocytes through inhibiting the expression of the identified specific lncRNAs, which is expected to be highly effective in the prevention or treatment of bone-related diseases.

## Description

### Technical Field

The present invention relates to a composition for preventing or treating osteoarthritis by modulating hypertrophic change in an articular chondrocyte and use thereof.

### Background Art

Osteoarthritis (OA), also known as degenerative arthritis, is a condition in which the articular cartilage that lines the articular surfaces of bones wears away, exposing the bone beneath the cartilage, and the synovial membrane around the joint becomes inflamed, resulting in pain and deformity. It is characterized by the actual loss of articular cartilage due to several changes that occur within the tissue, such as dysfunction of chondrocytes and inflammation of the synovial tissue.

While there have been significant advances in the field of gene therapy for the control of joint inflammation and the regeneration of damaged articular cartilage using cell therapy, there is still a lack of research to control or prevent hypertrophic changes in an articular chondrocyte to treat osteoarthritis, where hypertrophic chondrocytes are associated with the destruction of articular cartilage and localized calcification.

Among long non-coding RNAs (lncRNAs), which are important regulators of various biological processes, the inventors identified two important lncRNAs that play a role in hypertrophic changes in normal chondrocytes through RNA sequencing and found that they are not only required for stabilization of the RUNX2 protein, but are also involved in hypertrophic changes during chondrogenic differentiation of mesenchymal stem cells (MSCs), leading to the invention.

### DISCLOSURE

### Technical Problem

An object of the present invention is to provide a composition for the diagnosis of bone-related disease.

Another object of the present invention is to provide a kit for the diagnosis of bone-related disease.

Still another object of the present invention is to provide a method for providing information for the diagnosis of bone-related disease.

Yet another object of the present invention is to provide a device for diagnosing bone-related disease.

Still yet another object of the present invention is to provide a composition that inhibits chondrocyte hypertrophy.

A further object of the present invention is to provide a method for inhibiting chondrocyte hypertrophy.

Another further object of the present invention is to provide a composition for preventing or treating bone-related disease.

Still another further object of the present invention is to provide a method for preventing or treating bone-related disease.

Yet another further object of the present invention is to provide a method for screening therapeutics for bone-related disease.

However, the technical challenges of the present invention are not limited to those mentioned above, and other challenges not mentioned will become apparent to one of ordinary skill in the art from the following description.

### Technical Solution

Hereinafter, various embodiments described herein will be described with reference to the drawings. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order not to unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise defined in the specification, all the scientific and technical terms used in the specification have the same meanings as commonly understood by those skilled in the technical field to which the present invention pertains

One embodiment of the present invention is directed to a composition for diagnosing bone-related disease.

In the present invention, the diagnostic composition may comprise an agent for measuring the expression level of long non-coding RNAs (lncRNAs).

In the present invention, long non-coding RNAs (lncRNAs) are one type of RNA, defined as transcripts of 200 nucleotides or more that are not normally translated into proteins, and are characterized by their structural features and subcellular localization. While RNA sequencing has uncovered a large number of transcriptomes, little is known about the role and function of long non-coding RNAs.

In the present invention, the long non-coding RNA (lncRNA) may comprise at least one selected from the group consisting of LINC02035, LOC100130207, LINC02593, LOC100287808, LMNTD2-AS1, HCG4B, and LOC100287175, and more particularly may comprise LINC02035 or LOC100130207, but is not limited to.

In the present invention, LINC02035 (Long Intergenic Non-Protein Coding RNA 2035) corresponds to an RNA gene belonging to the class of lncRNA and may be represented by SEQ ID NO: 1, but is not limited to.

In the present invention, LOC100130207 corresponds to an RNA gene belonging to the lncRNA class, and may be represented by SEQ ID NO: 2, but is not limited to.

In the present invention, LINC02593 (Long Intergenic Non-Protein Coding RNA 2593) corresponds to an RNA gene belonging to the class of lncRNA and may be, but is not limited to, that represented by SEQ ID NO: 3.

In the present invention, LOC100287808 may be an RNA gene belonging to the ncRNA class and represented by SEQ ID NO: 4, but is not limited to.

In the present invention, LMNTD2-AS1 (LMNTD2 Antisense RNA 1) corresponds to an RNA gene belonging to the lncRNA class and may be the one represented by SEQ ID NO: 5, but is not limited to.

In the present invention, HCG4B (HLA Complex Group 4B) corresponds to an RNA gene belonging to the lncRNA class, and may be the one represented by SEQ ID NO: 6, but is not limited to.

In the present invention, LOC100287175 may be an RNA gene belonging to the lncRNA class and represented by SEQ ID NO: 7, but is not limited to.

An agent for measuring the expression level of the lncRNA of the present invention may comprise, but is not limited to, at least one selected from the group consisting of primers, probes and antisense nucleic acids that specifically bind to the lncRNA.

In the present invention, a "primer" is a fragment that recognizes a target gene sequence and includes a forward and reverse primer pair, preferably a primer pair that provides an assay result with specificity and sensitivity. High specificity may be conferred when the nucleic acid sequence of the primer is a sequence that is mismatched to a non-target sequence present in the sample, such that the primer amplifies only target gene sequences containing complementary primer binding sites and does not cause non-specific amplification.

In the present invention, the term "probe" refers to a substance that can specifically bind to a target substance to be detected in a sample, and through said binding, the presence of the target substance in the sample can be specifically identified. The type of probe may be any substance conventionally used in the art, but is preferably a peptide nucleic acid (PNA), locked nucleic acid (LNA), peptide, polypeptide, protein, RNA, or DNA, most preferably a PNA. More specifically, the probe may be a biomaterial, including one derived from or similar to a living organism or manufactured ex vivo, for example, an enzyme, a protein, an antibody, a microorganism, an animal or plant cell or organ, a nerve cell, DNA, and RNA, wherein DNA includes cDNA, genomic DNA, oligonucleotides, RNA includes genomic RNA, mRNA, oligonucleotides, and examples of proteins may include antibodies, antigens, enzymes, peptides, and the like.

As used herein, the term "locked nucleic acids (LNA)" refers to nucleic acid analogs comprising a 2'-O, 4'-C methylene bridge [J Weiler, J Hunziker and J Hall Gene Therapy (2006) 13, 496.502]. LNA nucleosides contain the common nucleic acid bases of DNA and RNA, and can form base pairs according to the Watson-Crick base pairing rule. However, due to the "locking" of the molecule due to the methylene bridge, LNA does not form the ideal conformation in Watson-Crick bonds. When LNA is incorporated into DNA or RNA oligonucleotides, it can pair more rapidly with complementary nucleotide chains, increasing the stability of the double helix.

As used herein, "antisense" refers to an oligomer having a sequence of nucleotide bases and an inter-subunit backbone that allows the antisense oligomer to hybridize to a target sequence in RNA by Watson-Crick base-pairing, thereby permitting the formation of an mRNA and RNA oligomeric heterodimer, typically within the target sequence. The oligomers can have exact sequence complementarity or approximate sequence complementarity to the target sequence.

Since the according to the present invention are known, those skilled in the art will be able to use them to facilitate the design of primers, probes, or antisense nucleotides that specifically bind to said lncRNAs.

The diagnostic composition of the present invention can diagnose a bone-related disease by comparing the expression level of lncRNA measured in a biological sample isolated from a subject to a control to determine whether the expression level is increased or decreased.

In the present invention, the term "diagnosis" refers to the identification of the presence or characterization of a pathological condition, and more specifically to the determination of a bone-related disease, in particular osteoarthritis, which is caused by dysregulated hypertrophy of chondrocytes.

In the present invention, bone-related diseases comprise osteoarthritis, rheumatoid arthritis, osteoporosis, osteoporosis, osteomalacia, osteopenia, osteopetrosis, bone atrophy, fibrous dysplasia, Paget's disease, and hypercalcemia, giant cell tumor, neoplastic destruction of bone, cancer-associated bone resorption disease, fracture, osteolysis, and chondrocalcinosis, which may be one or more selected from the group consisting of, but is not limited to, diseases caused by dysregulated hypertrophy of chondrocytes.

In the present invention, arthritis is an inflammation of the joints, which are composed of cartilage, joint capsule, synovial membrane, ligaments, tendons, muscles, etc. that allow smooth movement between bones and bones, and is broadly categorized into osteoarthritis (degenerative arthritis) and rheumatoid arthritis. Osteoarthritis (degenerative arthritis) is a localized degenerative change in joint cartilage, which can be caused by overuse of the cartilage over a long period of time, such as repetitive work or exercise, obesity, joint strain, or joint trauma. Rheumatoid arthritis is a chronic inflammatory systemic disease characterized by a persistent inflammatory response in the synovial membrane of the joints. The exact cause is not known, but it is hypothesized to be an immune response in which individuals with a genetic predisposition are exposed to environmental factors such as smoking or infectious agents such as periodontitis, which trigger the production of autoantibodies and an inflammatory response.

Chondrocyte hypertrophy is one of the hallmarks of osteoarthritis, and chondrocytes undergoing hypertrophic changes are known to be involved in tissue remodeling and calcification. Articular cartilage is broadly divided into two layers: calcified and non-calcified, with the non-calcified region of cartilage containing the surface area that can determine the dynamic nature of tissue homeostasis. In general, chondrocytes present in the non-calcified zone release cartilage matrix proteins such as COL2A1 and ACGN, whereas chondrocytes present in the calcified zone exhibit a hypertrophic morphology that is distinguished by the expression of RUNX2 and COL10A14. However, chondrocytes in non-calcified areas can be positive for hypertrophic markers such as RUNX2, COL10A1, and MMP13 in the osteoarthritic setting, suggesting that hypertrophic changes in chondrocytes present in non-calcified areas are strongly implicated in osteoarthritis progression, and thus hypertrophic differentiation of chondrocytes within the superficial region of articular cartilage may be one of the major phenotypic changes in osteoarthritis development. Furthermore, several factors such as transcription factors, cytokines, and matrix proteins are involved in chondrocyte hypertrophy. In particular, RUNX2 is a key regulator of chondrocyte hypertrophy and bone metabolism, and RUNX2 overexpression is known to induce hypertrophic differentiation of hypertrophic chondrocytes.

For the purposes of the present invention, the composition for the diagnosis of bone-related disease is more effective in the diagnosis of bone-related diseases, in particular osteoarthritis, as they involve the regulation of chondrocyte hypertrophy with a novel target involving RUNX2, unlike those previously reported.

Another embodiment of the present invention relates to a kit for the diagnosis of bone-related diseases comprising a composition of the present invention.

The kits of the present invention can be used to predict the presence of a bone-related disease, particularly osteoarthritis, in a subject using the compositions of the present invention if the lncRNA is present at higher levels in the subject compared to controls. More specifically, the presence of expression levels of at least one selected from the group consisting of LINC02035, LOC100130207, LINC02593, LOC100287808, LMNTD2-AS1, HCG4B, and LOC100287175 at higher levels compared to controls is predictive of the presence of a bone-related disease, in particular osteoarthritis, due to abnormalities in the regulation of hypertrophy of chondrocytes. More specifically, a subject having an expression level of at least one of LINC02035 or LOC100130207 at a higher level compared to a control is predictive of having a bone-related disease, in particular osteoarthritis, due to an abnormality in the regulation of chondrocyte hypertrophy.

In the present invention, the "control" may be a normal control, more specifically, it may be obtained from a serum sample of a patient confirmed to be free of bone-related disease, or it may be an average to median value of the expression level of the lncRNA in a patient who has undergone bone density testing, ultrasound and CT and who is finally confirmed to be free of bone-related disease. The expression level of the marker protein or gene encoding the lncRNA in a control group may be compared to the expression level of the marker protein or gene encoding the lncRNA in a biological sample from a patient with the bone-related disease being analyzed, and a diagnosis of the bone-related disease may be made by determining whether there is a significant change in the expression level.

As used herein, "high level" means measurably and significantly elevated expression relative to a normal control, and more particularly means, for example, at least 20% higher expression relative to a normal control, more particularly at least 30% higher expression, more particularly at least 40% higher expression, and most particularly at least 50% higher expression, but is not limited thereto.

In the above diagnostic kit of the present invention, the description of lncRNA, bone-related diseases, etc. is the same as in the diagnostic compositions, and is omitted to avoid undue complexity of the present description.

The kit of the present invention may be, but are not limited to, RT-PCR kits or DNA chip kits.

The kits of the present invention may further comprise one or more other component compositions, solutions, or devices suitable for the assay method. For example, the kits of the present invention may further comprise essential elements necessary to perform a reverse transcription polymerase reaction. The reverse transcription polymerase reaction kit comprises a pair of primers specific for a gene encoding a marker protein. The primers are nucleotides having a sequence specific to the nucleic acid sequence of said gene, and may have a length of from about 7 bp to 50 bp, more preferably from about 10 bp to 30 bp. It can also include a primer specific for a nucleic acid sequence of a control gene. Other reverse transcription polymerase reaction kits may include a test tube or other suitable container, reaction buffer (pH and magnesium concentration vary), deoxynucleotides (dNTPs), enzymes such as Taq-polymerase and reverse transcriptase, DNase, RNase inhibitor DEPC-water, sterile water, and the like.

Furthermore, the diagnostic kits of the present invention may include the essential elements required to perform a DNA chip. A DNA chip kit can include a substrate to which a cDNA or oligonucleotide corresponding to a gene or fragment thereof is attached, and reagents, agents, enzymes, etc. for making a fluorescently labeled probe. The substrate can also include a cDNA or oligonucleotide corresponding to a control gene or a fragment thereof.

Still another embodiment of the present invention is directed to a method of providing information for diagnosing a bone-related condition.

The method of the present invention may comprise measuring the expression level of long non-coding RNAs (lncRNA) in a biological sample isolated from a subject of interest.

In the present invention, the lncRNA may comprise at least one selected from the group consisting of LINC02035, LOC100130207, LINC02593, LOC100287808, LMNTD2-AS1, HCG4B, and LOC100287175, and more particularly may comprise LINC02035 or LOC100130207, but is not limited thereto.

The methods of the present invention may be for screening for the presence or absence of bone-related disease, in particular osteoarthritis, in a biological sample isolated from a subject of interest.

As used in the present invention, the "subject of interest" is a mammal, comprising a human, which may be selected from the group consisting of, for example, humans, rats, mice, marmots, hamsters, rabbits, monkeys, dogs, cats, cows, horses, pigs, sheep, and goats, and more particularly, but not exclusively, humans.

As used herein, the term "human" may refer to, but is not limited to, a patient who has developed or is suspected of developing a bone-related disease and who requires or is expected to require appropriate treatment of a bone-related disease, in particular osteoarthritis, based on dysregulation of chondrocyte hypertrophy.

The term "biological sample" in the present invention refers to any material, biological fluid, tissue, or cell obtained from or derived from the subject who is a patient who has developed a bone-related disease or is suspected of developing a bone-related disease and has a suspected dysregulation of chondrocyte hypertrophy, For example, blood, including whole blood, leukocytes, peripheral blood mononuclear cells, leukocyte buffy coat, plasma, and serum; sputum; tears; mucus; nasal washes; and nasal aspirate, breath, urine, semen, saliva, peritoneal washes, pelvic fluids, cystic fluid, cerebrospinal fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, or cerebrospinal fluid.

Measuring the expression level of the lncRNA of the present invention may be performed by, but is not limited to, reverse transcription polymerase reaction (RT-PCR), competitive RT-PCR, real-time RT-PCR, RNase protection assay (RPA), Northern blotting, or DNA chip using said composition of the present invention.

In the above methods of providing diagnostic information of the present invention, descriptions of lncRNA, agents for measuring expression levels, bone-related diseases, hypertrophy of chondrocytes, and the like are the same as those described for the diagnostic compositions and are omitted to avoid undue complexity of the present description.

The method of the present invention can predict that a subject of interest has a bone-related disease if the expression level of lncRNA measured in said biological sample is higher than a normal control. More specifically, where the expression level of at least one lncRNA selected from the group consisting of LINC02035, LOC100130207, LINC02593, LOC100287808, LMNTD2-AS1, HCG4B, and LOC100287175 is higher than a normal control, the subject can be predicted to have a bone-related disease, in particular osteoarthritis, caused by dysregulation of hypertrophy of chondrocytes. More specifically, a subject having an expression level of at least one of LINC02035 or LOC100130207 at a higher level than a normal control is predictive of having a bone-related disease, in particular osteoarthritis, due to dysregulation of hypertrophy of chondrocytes.

Yet another embodiment of the present invention is directed to a diagnostic device for a bone-related disease.

The measurement unit of the diagnostic device of the present invention may measure the expression level of lncRNA using an agent that measures the expression level of lncRNA in a biological sample obtained from a subject.

The subject of interest of the present invention may be selected from the group consisting of humans, rats, mice, marmots, hamsters, rabbits, monkeys, dogs, cats, cattle, horses, pigs, sheep, and goats, and may be specifically human, but is not limited thereto.

In the present invention, the biological samples include whole blood, leukocytes, peripheral blood mononuclear cells, leukocyte buffy coat, plasma, serum, sputum, tears, mucus, nasal washes, nasal aspirate, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cell, cell extract, and tissue, and may be one or more species selected from the group consisting of, but not limited to, the following.

The agent utilized by the measurement unit of the diagnostic device of the present invention may be an agent that measures the expression level of lncRNA. More specifically, it may comprise at least one selected from the group consisting of primers, probes and antisense nucleotides that specifically bind to said gene.

By determining the degree of expression of the genes using the agents in the measuring unit of the diagnostic device of the present invention, it is possible to predict the presence or absence of bone-related diseases, more specifically bone-related diseases caused by abnormalities in the regulation of hypertrophy in chondrocytes, and in particular osteoarthritis.

The diagnostic device for a bone-related disease of the present invention may further comprise a detection unit for predicting and outputting the presence or absence of a bone-related disease, in particular osteoarthritis, of a objective subject from the degree of expression of the gene obtained from the measurement unit.

In the present invention, the detection unit can diagnose a bone-related disease, in particular osteoarthritis, by generating and categorizing information that a bone-related disease caused by dysregulation of hypertrophy of chondrocytes has developed or is likely to develop if the expression level of the gene obtained from the measurement unit is higher than the expression level of the lncRNA measured in a control.

Furthermore, in the present invention, said detection part can diagnose a bone-related disease by generating information and categorizing the patient as having developed or likely to develop a bone-related disease, in particular osteoarthritis due to hypertrophy of chondrocytes, if the expression level of said gene obtained from said measurement part is higher than the expression level of the lncRNA measured in the control group.

Bone-related diseases in the diagnostic device of the invention comprise osteoarthritis, rheumatoid arthritis, osteoporosis, osteomalacia, osteopenia, osteopetrosis, bone atrophy, fibrotic dysplasia, osteopenia, osteopetrosis, bone atrophy, fibrous dysplasia, Paget's disease, and hypercalcemia, giant cell tumor, neoplastic destruction of bone, cancer-associated bone resorption disease, fracture, osteolysis, and chondrocalcinosis, which may be one or more selected from the group consisting of, but is not limited to, diseases caused by dysregulated hypertrophy of chondrocytes.

Still yet another embodiment of the invention, the present invention is directed to a composition that inhibits hypertrophy of chondrocytes.

The composition of the present invention may comprise an agent that reduces the expression level of long non-coding RNAs (lncRNA).

In the present invention, the lncRNA may be at least one selected from the group consisting of LINC02035, LOC100130207, LINC02593, LOC100287808, LMNTD2-AS1, HCG4B, and LOC100287175, and more particularly may be at least one of LINC02035 or LOC100130207, but is not limited thereto.

The agent for reducing the expression of said lncRNA of the present invention may comprise an antisense nucleotide that binds complementarily to at least one selected from the group consisting of the lncRNA gene, more particularly LINC02035, LOC100130207, LINC02593, LOC100287808, LMNTD2-AS1, HCG4B and LOC100287175, an antisense nucleotide, a small interfering RNA (short interfering RNA; siRNA), short hairpin RNA, and a ribozyme, but may include, without limitation, any one or more selected from the group consisting of that directly or indirectly act on a target protein or gene to produce an effect in which the activity or expression thereof is inhibited, provided that the effect is readily derivable by methods known in the art.

In the present invention, an "antisense nucleotide" is one that binds (hybridizes) to a complementary sequence in DNA, immature mRNA, or mature mRNA, as defined by Watson-Crick base pairing, and disrupts the flow of genetic information from DNA to protein. The specificity of antisense nucleotides to their target sequences makes them exceptionally multifunctional. Because antisense nucleotides are long chains of monomeric units, they can be easily synthesized against target RNA sequences. Many recent studies have demonstrated the utility of antisense nucleotides as a biochemical tool for studying target proteins. Since there have been many recent advances in oligonucleotide chemistry and the synthesis of nucleotides that exhibit improved cell line uptake, target binding affinity, and nuclease resistance, the use of antisense nucleotides can be considered as a new form of inhibitor.

In the present invention, "siRNA" and "shRNA" are nucleic acid molecules that can mediate RNA interference or gene silencing, and are used as efficient gene knockdown methods or gene therapy methods because they can inhibit the expression of target genes. In vivo, shRNA is a single-stranded oligonucleotide that is formed into a hairpin structure by the binding of complementary sequences within a single-stranded oligonucleotide, and upon cleavage by a dicer, the shRNA becomes a small RNA fragment of 21 to 25 nucleotides in size, which is a double-stranded oligonucleotide, siRNA, which can specifically bind to mRNA with complementary sequences and inhibit its expression. Thus, the choice of whether to utilize shRNA or siRNA can be determined by those skilled in the art, and so long as the mRNA sequences they target are identical, similar expression reduction effects can be expected. For the purposes of the present invention, the expression of said lncRNA can be inhibited by acting specifically on said lncRNA to cleave the gene (e.g., mRNA molecule) of said lncRNA, thereby inducing the phenomenon of RNA interference (RNAi). The siRNA can be synthesized chemically or enzymatically. The method of preparation of the siRNA is not particularly limited, and any method known in the art can be used. Examples include, but are not limited to, direct chemical synthesis of siRNA, synthesis of siRNA by in vitro transcription, enzymatic cleavage of long double-stranded RNA synthesized by in vitro transcription, expression by intracellular delivery of shRNA expression plasmids or viral vectors, and expression by intracellular delivery of polymerase chain reaction (PCR)-directed siRNA expression cassettes.

As used herein, the term "ribozyme" refers to an RNA molecule having catalytic activity. Ribozymes with various activities are known in the art, and ribozymes of lncRNA genes include known or artificially generated ribozymes, and optionally ribozymes with target-specific RNA cleavage activity can be prepared by standard techniques in the art.

The composition of the present invention is applicable to the prevention or treatment of bone-related diseases in which dysregulation of chondrocyte hypertrophy or calcification is predicted by inhibiting chondrocyte hypertrophy.

In the above compositions for inhibiting chondrocyte hypertrophy of the present invention, the description of lncRNAs, bone-related diseases, etc. is the same as in the compositions for diagnostic use, and is omitted to avoid undue complexity of the present description.

A further embodiment of the present invention, a method of inhibiting hypertrophy of chondrocytes is directed to a subject in need thereof, comprising administering to the subject in need thereof an effective amount of a composition for inhibiting hypertrophy of chondrocytes comprising an agent that reduces the expression level of long non-coding RNAs (lncRNA).

Descriptions of the agent that reduce the expression levels of lncRNA, long non-coding RNAs (lncRNA), hypertrophy of chondrocytes, and the like in the methods of the present invention are identical to those previously described and are omitted to avoid undue complexity of the present description.

Another further embodiment of the present invention is directed to a pharmaceutical composition for the prevention or treatment of bone-related diseases that inhibits hypertrophy or calcification of chondrocytes.

The pharmaceutical compositions of the present invention may comprise an agent that reduces the expression levels of long non-coding RNAs (lncRNAs).

In the present invention, said lncRNA may be at least one selected from the group consisting of LINC02035, LOC100130207, LINC02593, LOC100287808, LMNTD2-AS1, HCG4B, and LOC100287175, and more particularly may be at least one of LINC02035 or LOC100130207, but is not limited thereto.

In the present invention, the pharmaceutical composition may treat or prevent bone-related diseases, in particular osteoarthritis, by reducing the expression level of said lncRNA, thereby inhibiting hypertrophy or calcification of chondrocytes.

The pharmaceutical composition of the present invention has the function of preventing, ameliorating, or treating a bone-related disease by inhibiting hypertrophy or calcification of chondrocytes, wherein the bone-related disease is osteoarthritis, rheumatoid arthritis, osteoporosis, osteomalacia, osteopenia, osteopetrosis, bone atrophy, fibrous dysplasia, and Paget's disease, hypercalcemia, giant cell tumor, neoplastic destruction of bone, cancer-related bone resorption disease, fracture, osteolysis, and chondrocalcinosis, and may be one or more selected from the group consisting of, but is not limited to, osteoarthritis, and may be specifically osteoarthritis, provided that it is a disease caused by dysregulated hypertrophy of chondrocytes.

Furthermore, the pharmaceutical composition is applicable to the prevention or treatment of bone-related diseases, in particular osteoarthritis, in which dysregulated hypertrophy of chondrocytes ultimately leads to calcification.

As used herein, the term "preventing" may include, without limitation, any act by which a composition of the present invention can be used to block symptoms caused by a bone-related disease, or to inhibit or delay progression to osteoarticular disease.

As used in the present invention, the term "treatment" refers to a set of activities performed to alleviate and/or ameliorate a disease of interest. For the purposes of the present invention, treatment includes any activity that inhibits or delays bone-related disease, in particular osteoarthritis, and may include, without limitation, any activity that ameliorates or benefits symptoms caused by bone-related disease, in particular osteoarthritis.

In the pharmaceutical composition for the prevention or treatment of bone-related disease of the present invention, the description of lncRNA, bone-related disease, osteoarthritis, and the like is the same as described in the diagnostic compositions and the compositions for inhibiting chondrocyte hypertrophy, and is omitted to avoid undue complexity of the description.

The pharmaceutical composition of the invention may be characterized by being in the form of capsules, tablets, granules, injections, ointments, powders or beverages, and said pharmaceutical composition may be characterized by being intended for human use.

The pharmaceutical composition of the present invention is not limited to, but may be formulated and used in the form of oral dosage forms such as pills, granules, capsules, tablets, aqueous suspensions, topicals, suppositories, and sterile injection solutions, respectively, according to methods known in the art. The pharmaceutical compositions of the present invention may comprise a pharmaceutically acceptable carrier. Pharmaceutically acceptable carriers may include binders, activators, disintegrants, excipients, solubilizers, dispersants, stabilizers, suspending agents, colors, flavors, and the like for oral administration; buffers, preservatives, currency-free agents, solubilizers, isotonic agents, stabilizers, and the like for injectable formulations; and carriers, excipients, lubricants, preservatives, and the like for topical application. Formulations of the pharmaceutical compositions of the present invention can be prepared in a variety of ways by mixing with pharmaceutically acceptable carriers as described above. For example, for oral administration, they can be formulated as tablets, troches, capsules, elixirs, suspensions, syrups, wafers, and the like, and for injection, they can be formulated as unit dose ampoules or multiple dose forms. In addition, it can be formulated as a solution, suspension, tablet, capsule, extended-release formulation, etc.

Examples of carriers, excipients and diluents suitable for formulation include lactose, dextrose, sucrose, sorbitol, mannitol, xylitol, erythritol, malditol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, or mineral oil. It may further include fillers, anti-flocculants, lubricants, wetting agents, flavors, emulsifiers, preservatives, and the like.

Routes of administration of the pharmaceutical compositions of the present invention include, but are not limited to, oral, intravenous, intramuscular, intra-arterial, intramedullary, intrathecal, intracardiac, transdermal, subcutaneous, intraperitoneal, intranasal, intestinal, topical, sublingual, intra-articular or rectal. Parenteral administration, more particularly intra-articular administration, is preferred.

The term "parenteral" in the present invention includes subcutaneous, intradermal, intravenous, intramuscular, intra-articular, intra-bursal, intra-synovial, intrasternal, intradural, intra-lesional and intracranial injection or infusion techniques. Preferably, the pharmaceutical composition of the present invention may also be administered in the form of suppositories for rectal administration, but is not limited thereto.

The pharmaceutical composition of the present invention may vary depending on various factors, including the activity of specific compounds used, the patient's age, body weight, general health, sex, and diet, the period of administration, the route of administration, excretion rate, the drug content, and the severity of a specific disease to be prevented or treated. The dose of the pharmaceutical composition may be suitably selected by a person skilled in the art depending on the patient's condition and body weight, the severity of the disease, the form of drug, and the route and period of administration, and may be 0.0001 to 50 mg/kg/day or 0.001 to 50 mg/kg/day. The pharmaceutical composition may be administered once or several times a day. The dose is not intended to limit the scope of the present invention in any way. The pharmaceutical composition according to the present invention may be formulated as pills, sugar-coated tablets, capsules, liquids, gels, syrups, slurries, or suspensions.

Yet another further embodiment of the present invention is directed to a method of treating a bone-related disease comprising administering to a subject in need thereof an effective amount of a pharmaceutical composition for the prevention or treatment of a bone-related disease comprising an agent that reduces the expression level of long non-coding RNAs (lncRNA).

As used in the present invention, "administering" means providing a subject with a predetermined composition of the invention by any suitable method.

In the present invention, a "subject" in need of said administration may comprise both mammals and non-mammals. Examples of mammals may include, but are not limited to, humans, non-human primates, such as chimpanzees, other ape or monkey species; livestock animals, such as cattle, horses, sheep, goats, pigs; domesticated animals, such as rabbits, dogs or cats; laboratory animals, such as rodents, such as rats, mice or guinea pigs; and, examples of non-mammals in the present invention may include, but are not limited to, birds or fish.

The formulations of the compositions administered as described above in the present invention are not particularly limited and may be administered as solid dosage forms, liquid dosage forms, or aerosol formulations for inhalation, and may be administered as solid dosage forms intended to be converted to liquid dosage forms for oral or parenteral administration immediately prior to use, and may be administered in the form of oral dosage forms, such as, but not limited to, granules, capsules, tablets, aqueous suspensions, topicals, suppositories, and sterile injectable solutions.

Furthermore, in the present invention, a pharmaceutically acceptable carrier may be further administered with the composition of the invention upon said administration. The pharmaceutically acceptable carrier can be a binder, an active agent, a disintegrant, an excipient, a solubilizer, a dispersant, a stabilizer, a suspending agent, a color, a flavor, or the like for oral administration; a buffer, a preservative, a currency-free agent, a solubilizer, an isotonic agent, a stabilizer, or the like for injectable administration; or a base, an excipient, a lubricant, a preservative, or the like for topical administration. Formulations of the compositions of the present invention can be prepared in a variety of ways by mixing with pharmaceutically acceptable carriers as described above. For example, for oral administration, they can be prepared in the form of tablets, troches, capsules, elixirs, suspensions, syrups, wafers, and the like, and for injection, they can be prepared in the form of unit dose ampoules or multiple dose forms. Other formulations include solutions, suspensions, tablets, capsules, extended-release preparations, etc.

Examples of carriers, excipients and diluents suitable for formulation include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, or mineral oil. It may further include fillers, anti-flocculants, lubricants, wetting agents, flavors, emulsifiers, preservatives, and the like.

Routes of administration of the compositions according to the invention include, but are not limited to, oral, intravenous, intramuscular, intra-arterial, intramedullary, intrathecal, intracardiac, transdermal, subcutaneous, intraperitoneal, intranasal, enteral, topical, sublingual or rectal. Oral or parenteral administration is preferred.

As used herein, "parenteral" includes subcutaneous, intradermal, intravenous, intramuscular, intra-articular, intra-synovial, intrasternal, intradural, intra-lesional and intra-cranial injection or infusion techniques. The pharmaceutical composition of the present invention may also be administered in the form of suppositories for rectal administration.

In the present invention, a "pharmaceutically effective amount" refers to a sufficient amount of an agent to provide a desirable biological result. The result may be a reduction and/or alleviation of a sign, symptom, or cause of a disease, or any other desirable change in an organism. For example, an "effective amount" for therapeutic use is an amount of a composition disclosed herein that is required to provide a clinically significant reduction in a disease. An appropriate "effective" amount in any individual case can be determined by those skilled in the art using routine experimentation. Thus, the expression "effective amount" generally refers to an amount in which the active substance has a therapeutic effect. In the case of the present invention, the active substance is an agent for inhibiting chondrocyte hypertrophy, and for the prevention, amelioration or treatment of bone-related disorders.

The composition of the present invention may be varied depending on a number of factors, including the activity of the particular agent used, age, weight, general health, gender, formulation, time of administration, route of administration, elimination rate, drug formulation, and severity of the particular disease to be prevented or treated, and the dosage of the composition may be suitably selected by those skilled in the art, depending on the patient's condition, weight, severity of the disease, drug form, route of administration, and duration, but may be administered in an amount from 0.0001 to 100 mg/kg or 0.001 to 100 mg/kg per day. The dose may be administered once daily or may be divided into multiple doses. The above dosages are not intended to limit the scope of the invention in any way. The compositions according to the invention can be formulated as pills, tablets, capsules, liquids, gels, syrups, slurries, suspensions, and the like.

Still yet another further embodiment of the present invention is directed to a method for screening a pharmaceutical composition for bone-related disease.

The method of screening of the present invention may comprise: bringing a candidate substance into contact with a biological sample isolated from a subject of interest; and measuring the expression level of at least one long non-coding RNAs (lncRNA) selected from the group consisting of LINC02035, LOC100130207, LINC02593, LOC100287808, LMNTD2-AS1, HCG4B, and LOC100287175 in the biological sample.

In the above screening method of the present invention, the descriptions of lncRNA, bone-related disease, and the like are the same as those described in the diagnostic compositions, and are omitted to avoid undue complexity of the present description.

The candidate substance of the present invention refer to agents for testing whether they have inhibitory activity for overcoming bone-related diseases, in particular osteoarthritis, i.e., whether they have the activity to reduce the expression level of an lncRNA to a level equivalent to, or comparable to, the expression level in normal humans without bone-related diseases, in particular osteoarthritis, and include any molecule, such as proteins, oligopeptides, organic molecules, polysaccharides, polynucleotides, and a wide range of compounds. Such candidates may include both natural and synthetic substances.

The method of screening of the present invention may further comprise determining as the composition for treating the bone-related disease if the expression level of the lncRNA measured after treatment with the candidate substance decreased than those of the lncRNA measured before treatment with that.

### Advantageous Effects

The present invention relates to novel long non-coding RNAs (lncRNA) involved in the regulation of hypertrophy of chondrocytes, and is highly effective in the prevention or treatment of bone-related diseases because it is possible to diagnose bone-related diseases by measuring the degree of expression of certain discovered lncRNAs, and furthermore, by inhibiting the expression of certain discovered lncRNAs, it is possible to effectively inhibit the vascularization or calcification of articular cartilage by inducing the inhibition of hypertrophy of chondrocytes.

### Brief Description of Drawings

FIG. 1a is a diagram illustrating the role of RUNX2 in chondrocyte hypertrophy and osteoarthritis progression, in accordance with one embodiment of the present invention.
FIG. 1b is a diagram showing the results of Safranin O (SFO) staining and immunostaining of cartilage isolated from an osteoarthritis patient in accordance with one embodiment of the present invention.
FIGS. 2a and 2b show the results of qRT-PCR analysis of IHH, RUNX2, COL10A1, and PTGES mRNA expression and Western blot analysis of COX2, IHH, RUNX2, and COL10A1 protein levels in TC28a2 cells treated or untreated with inflammatory cytokines (10 ng/ml IL-1β, 50 ng/ml IL-6, or 10 ng/ml TNF-α), in accordance with one embodiment of the present invention.
FIGS. 3a and 3b show the results of qRT-PCR analysis of IHH, RUNX2, COL10A1, and BGLAP mRNA expression and western blot analysis of protein levels of COX2, IHH, RUNX2, and COL10A1 in TC28a2 cells in the presence and absence of hypertrophic medium, in accordance with one embodiment of the present invention.
Figure 4 shows the results of western blot analysis of protein levels of RUNX2, IHH, COL10A1, COL2A1, and ACAN in TC28a2 cells transfected with siRNAs treated with or without hypertrophic medium/TNF-α (10 ng/ml) according to one embodiment of the present invention.
FIGS. 5a and 5b are fluorescence images showing the activity of an OCN promoter and the amount of OCN promoter reporter according to one embodiment of the present invention, and a bar graph showing the fluorescence-based activity of the OCN promoter.
FIGS. 6a to 6c are diagrams depicting heatmaps generated by hierarchical clustering of RNA sequencing data using RNA extracted from TC28a2 cells grown in growth medium (GM) or hypertrophic medium (HM), according to one embodiment of the present invention, and diagrams of associated cell signaling pathways based on Kegg pathway analysis.
FIG. 7 shows the results of a western blot analysis of protein levels of AKT, p-AKT, FOXO3A, and p-FOXO3A in TC28a2 cells treated with or without hypertrophic medium/TNF-α according to one embodiment of the present invention.
FIG. 8 is a representation of a volcano plot of differentially expressed RNAs between the GM and HM groups, in accordance with one embodiment of the present invention.
FIG. 9 is a heatmap of differentially expressed during hypertrophic differentiation of TC28a2 cells in accordance with one embodiment of the present invention.
FIG. 10a is a diagram showing the results of a western blot analysis of protein levels of FLAG and RUNX2 in TC28a2 cells transfected with a pcDNA-mock or pcDNA-RUNX2-FLAG plasmid vector according to one embodiment of the present invention.
FIG. 10b shows the results of a Western blot analysis of protein levels of RUNX2 using RNA-IP samples obtained from TC28a2 cells transfected with a pcDNA-RUNX2 vector according to one embodiment of the present invention.
FIG. 10c is a bar graph showing the results of RNA-IP in TC28a2 cells transfected with pcDNA-RUNX2 vector according to one embodiment of the present invention, and RNA-IP-qPCR fold enrichment of compared to IgG control.
FIG. 11a is a diagram showing the results of qRT-PCR analysis of each lncRNA expression in TC28a2 cells transfected with siRNA (100 nM) targeting each lncRNA according to one embodiment of the present invention.
FIG. 11b shows the results of western blot analysis of protein levels of RUNX2 in TC28a2 cells transfected with each siRNA with or without hypertrophic medium/TNF-α (10 ng/ml), according to one embodiment of the present invention.
FIG. 11c is a diagram of the intersection of groups in which transfection of TC28a2 cells with each siRNA according to one embodiment of the present invention did not change the amount of RUNX2 protein in both early and late stages despite treatment with hypertrophic medium or TNF-α.
FIG. 11d is a diagram showing the knockdown effect of LINC02035 or LOC100130207 on RUNX2 activity, according to one embodiment of the present invention, as determined by enzyme-linked immunosorbent assay (ELISA).
FIG. 12a shows the results of Western blot analysis of protein levels of IHH, COL10A1, and MMP13 in siRNA-transfected TC28a2 cells treated with or without hypertrophic medium/TNF-α (10 ng/ml) in accordance with one embodiment of the present invention.
FIGS. 12b and 12c are fluorescence images showing the activity of the OCN promoter and the amount of OCN promoter reporter, and a bar graph of the fluorescence-based activity of the OCN promoter, in accordance with one embodiment of the present invention.
FIG. 12d is a diagram showing the results of western blot analysis of protein levels of COL2A1 and ACAN in siRNA-transfected TC28a2 cells treated with or without hypertrophic medium/TNF-α (10 ng/ml) in accordance with one embodiment of the present invention.
FIG. 13 is a view of the results of RNA in situ hybridization performed on primary chondrocytes isolated from intact or damaged cartilage tissue obtained from an OA patient in accordance with one embodiment of the present invention.
FIG. 14 shows the distribution of LINC02035 and LOC100130207 in the cytoplasm and nucleus of TC28a2 cells treated with hypertrophic medium or TNF-α (10 ng/ml) according to one embodiment of the present invention.
FIG. 15 shows the results of a western blot analysis of protein levels of RUNX2 in TC28a2 cells treated or not treated with MG132 according to one embodiment of the present invention.
FIGS. 16a and 16b show the results of IP/Western blot analysis performed to investigate whether hypertrophic medium or TNF-α treatment in accordance with one embodiment of the present invention affects ubiquitination of RUNX2 protein in TC28a2 cells.
FIGS. 17a and 17b are diagrams showing the results of qRT-PCR analysis of SOX9, COL2A1, RUNX2, and COL10A1 mRNA and Western blot analysis of protein levels of SOX9, COL2A1, RUNX2, and COL10A1 during chondrogenic differentiation of human bone marrow-derived MSCs according to one embodiment of the present invention.
FIG. 17c is a diagram showing the results of qRT-PCR analysis of LINC02035 and LOC100130207 during chondrogenic differentiation of human bone marrow-derived MSCs according to one embodiment of the present invention.
FIG. 18a is a diagram showing the results of qRT-PCR analysis of LINC02035 and LOC100130207, RUNX2 and COL10A1 expression during chondrogenic differentiation of human bone marrow-derived MSCs transfected with each targeting siRNA according to one embodiment of the present invention.
FIG. 18b is a diagram showing the results of proteoglycan staining by Safranin O and Alcian blue and immunostaining for RUNX2 according to one embodiment of the present invention.
FIG. 18c is a diagram showing the results of Western blot analysis of protein levels of SOX9, COL2A1, RUNX2, and COL10A1 during chondrogenic differentiation of human bone marrow-derived MSCs transfected with siRNA targeting LINC02035 or LOC100130207 according to one embodiment of the present invention.
FIGS. 19a and 19b illustrate the transfection of human bone marrow-derived mesenchymal stem cells (hMSCs) with respective siRNAs (control, LINC02035 and LOC100130207) to induce chondrogenic differentiation and subsequent transplantation into nude mice for histological analysis in vivo according to one embodiment of the present invention.
FIG. 20 is a diagram showing the results of immunohistochemical staining for RUNX2 and IgG, along with Safranin O and H&E staining of isolated tissue from a mouse according to one embodiment of the present invention.

### Best Mode

One embodiment of the present invention is directed to a composition for the diagnosing a bone-related disease comprising an agent for measuring the expression level of long non-coding RNAs (lncRNA).

Another embodiment of the invention is directed to a kit for diagnosing a bone-related disease comprising a composition for diagnosis of a bone-related disease of the present invention.

Still another embodiment of the present invention is directed to a method of providing information for diagnosing a bone-related disease, comprising measuring expression level of long non-coding RNAs (lncRNA) in a biological sample isolated from a subject.

Yet another embodiment of the present invention is directed to a diagnostic device for a bone-related disease comprising (a) a measurement unit for measuring the expression level of long non-coding RNAs (lncRNA) in a biological sample obtained from a subject; and (b) a detection unit for outputting, from the expression level of lncRNA measured in the measurement unit, whether the subject has developed or is likely to develop a bone-related disease.

Still yet another object of the present invention is directed to a composition for inhibiting chondrocyte hypertrophy comprising an agent that reduces the expression levels of long non-coding RNAs (lncRNA).

A further object of the present invention is directed to a method of inhibiting chondrocyte hypertrophy, comprising administering to a subject in need thereof an effective amount of a composition for inhibiting chondrocyte hypertrophy comprising an agent that reduces the expression level of long non-coding RNAs (lncRNA).

Another further object of the present invention is directed to a pharmaceutical composition for the treatment of a bone-related disease comprising an agent that reduces the expression level of long non-coding RNAs (lncRNA).

Still another further object of the present invention is directed to a method of treating a bone-related disease comprising administering to a subject in need thereof an effective amount of a pharmaceutical composition for the prevention or treatment of a bone-related disease comprising an agent that reduces the expression level of long non-coding RNAs (lncRNA).

Yet another further object of the present invention is directed to a method of screening for a treatment for a bone-related disease comprising: bringing a candidate substance into contact with a biological sample isolated from a subject of interest; and measuring the level of expression of at least one long non-coding RNAs (lncRNA) selected from the group consisting of LINC02035, LOC100130207, LINC02593, LOC100287808, LMNTD2-AS1, HCG4B, and LOC100287175 in the biological sample.

### Mode for Invention

Hereinafter, the present invention will be described in further detail by examples. It would be obvious to those skilled in the art that these examples are intended to be more concretely illustrative and that the scope of the present invention as set forth in the appended claims is not limited to or by the examples.

### Example

### Experimental Methods

### Preparation of human cartilage tissue, primary chondrocytes, TC28a2 cells and MSCs

The inventors obtained cartilage tissue samples from three osteoarthritis patients who underwent total knee arthroplasty with the approval of the Institutional Review Board (IRB) of Yonsei University College of Medicine (2019-1374-002). To isolate primary chondrocytes, cartilage tissue was shaved from the articular surface under sterile conditions, minced into as many slices as possible, and washed three times with phosphate-buffered saline (PBS). The sliced tissues were then incubated in Dulbecco's Modified Eagle's high-glucose medium (DMEM-HG; Invitrogen) with 2% collagenase (Sigma-Aldrich, St. Louis, MO, USA) and 0.5% dispase (Sigma-Aldrich) for 4 h at 37°C and 5% CO₂ under sterile conditions. The cell suspension was then passed through a 40 µm cell filter to remove impurities, and cells were cultured in DMEM-HG medium containing 10% fetal bovine serum (FBS; Gibco, Grand Island, NY, USA) and 1% antibiotic-antifungal solution under 37°C and 5% CO₂ conditions. A normal human chondrogenic cell line (TC28a2) was purchased from Sigma-Aldrich and cultured under the same conditions as above. Human bone marrow-derived mesenchymal stem cells (MSCs) were obtained from the posterior iliac crest of three adults with the approval of the Yonsei University College of Medicine IRB (2017-0308-001). MSCs isolated from bone marrow were screened based on their ability to adhere to cell culture plates, and all cultured cells (98%) were found to be positive for CD90 and CD105 and negative for CD34 and CD45. MSCs were cultured in DMEM-low glucose (LG; Invitrogen) medium containing 10% FBS and 1% antibiotic-antifungal solution under 37°C and 5% CO₂ conditions.

### Reagents and differentiation methods

IL-1β (R&D Systems Inc., Minneapolis, MN, USA), TNF-α (R&D Systems Inc.), and IL-6 (Koma Biotech, Seoul, South Korea) were used to create an inflammatory environment for chondrocytes. IL-1β and TNF-α were used at a concentration of 10 ng/ml based on previously published studies, and IL-6 was used at a concentration of 50 ng/ml based on previous studies. MG-132 (proteasome inhibitor; Sigma-Aldrich) was used to determine whether RUNX2 is regulated at the post-translational level in normal chondrocytes. To induce hypertrophic differentiation of chondrocytes, we referred to the differentiation medium composition described in a paper published by another group (Yahara Y, Takemori H, Okada M et al. Pterosin B prevents chondrocyte hypertrophy and osteoarthritis in mice by inhibiting Sik3. Nat Commun 2016; 7:10959.). Briefly, TC28a2 cells were cultured in 1% FBS, 1% insulin-transferrin selenium-A (ITS, Invitrogen), 100 ng/ml BMP-2 (R&D Systems Inc.), 10 ng/ml GDF-5 (PeproTech, Rosemont, IL, USA), 1 µM 3,3,5-Triodo-L-tyronine (T3) (Sigma-aldrich), 50 µg/ml L(+)-ascorbic acid (Sigma-Aldrich), DMEM-HG supplemented with 10 nM dexamethasone (Sigma-Aldrich), 10 mM β-glycerophosphate (Sigma-Aldrich), and 1% antibiotic-antifungal solution. The hypertrophic medium was changed every 2 days for 5 days. Micromass culture method was used to induce in vitro chondrogenic differentiation of MSCs. MSCs were trypsinized and resuspended in DMEM-HG at a density of 1 x 10⁷ cells/ml. Cells were seeded as dots in the center of individual wells of 24-well plates at 1 x 10⁵ cells per well in a 10 µl volume. Cells were allowed to attach for 2 h in a cell culture incubator, followed by chondrogenic medium consisting of DMEM-HG supplemented with 1% antibiotic-antifungal solution, 1% insulin transferrin selenium-A (ITS; Invitrogen), 50 mg/ml ascorbic acid (Sigma-Aldrich), 40 µg/ml L-proline (Sigma-Aldrich), and 10 ng/ml TGF-b3 (R&D Systems Inc.). The chondrogenic medium was changed every 2 days for 14 days. Safranin O and alcian blue staining were performed to evaluate the chondrogenic potential and proteoglycan synthesis of MSCs. The chondrogenic pellets were washed with PBS and fixed in 10% formalin for about 24 hours. After fixation, the pellets were dehydrated in ethanol and the dehydrated pellets were paraffin-embedded and sectioned. Micromass sections were deparaffinized and stained with Safranin O (1% in acetic acid solution, Sigma-Aldrich) or Alcian blue (0.3% in 70% ethanol, Sigma-Aldrich) for 30 minutes, after which the stained pellet sections were mounted and measured under a microscope.

### IHC

Articular cartilage tissue was fixed in 10% formalin at room temperature, and formalin-fixed specimens were embedded in paraffin. Paraffin-embedded sections were deparaffinized, rehydrated, washed with PBS, and tissue sections were used to assess cartilage tissue disruption and detect chondrocyte hypertrophy-related marker proteins in human cartilage tissue. Prepared tissue samples were sliced into 4 µm thick sections and anti-IHH (Santa Cruz Biotechnology, Santa Cruz, CA, USA), anti-RUNX2 (Santa Cruz Biotechnology), or anti-COL10A1 (Santa Cruz Biotechnology) antibodies were used to detect IHH, RUNX2, or COL10A1 levels, respectively. To visualize each target protein in anti-IHH, anti-RUNX2 or anti-COL10A1 antibody-probed tissues, samples were stained with aminoethylcarbazole (AEC; Abcam, Cambridge, UK). Stained tissues were observed using a VS120 virtual microscope (Olympus, Tokyo, Japan) and images of sections were analyzed using the OlyVIA 2.5 program (Olympus)..

### qPCR

Total RNA was isolated using the RNeasy kit (Qiagen, Valencia, CA, USA) according to the manufacturer's instructions. qPCR was performed as previously published. Total RNA (1 µg of total RNA) was reverse transcribed using the Omniscript kit (Qiagen, Hilden, Germany). The primer sets used in this study were validated and purchased from Bioneer (Daejeon, South Korea). The primer sets used in this study are shown in Table 1 below. Gene expression changes were measured in triplicate (n = 3) and calculated as the mean value and normalized to the internal control ACTB (β-actin).

**[Table 1]**

| Genes | Primer sequence (5'→3') | SEQ ID NO |
|---|---|---|
| *IHH* | Cat. No- P101104 V (RNA accession: NM_002181.3) https://www.bioneer.co.kr/sirna-customorder.html | -. |
| *RUNX2* | Cat. No- P229954 V (RNA accession: NM_001015051.3)https://www.bioneer.co.kr/sirn a-customorder.html | -. |
| *COL10A1* | Cat. No- P261909 V (RNA accession: NM_000493.3)https://www.bioneer.co.kr/sirna-customorder.html | -. |
| *PTGES* | Cat. No- P327048 V (RNA accession: NM_004878.4) https://www.bioneer.co.kr/sirna-customorder.html | -. |
| *BGLAP* | Cat. No- P128146 V (RNA accession: NM_199173.5)https://www.bioneer.co.kr/sirna-customorder.html | -. |
| *LINC02593* | CCGAGCTTCAGAGGAGAGG (sense, NR_026874.2) | SEQ ID NO: 8 |
| | CTCCCAGATCGTGAGCTTTC (antisense) | SEQ ID NO: 9 |
| *IGFL2-AS1* | GGTGCCGAAGACCTGAGAC (sense, NR_135234.1) | SEQ ID NO: 10 |
| | GGCCACTTCTTCATTCTTGTTGG (antisense) | SEQ ID NO: 11 |
| *HCG4B* | GTCATTCACCGGCCTAGCTC (sense, NR_001317.3) | SEQ ID NO: 12 |
| | GAGTATTGGGACCGGAGCAC (antisense) | SEQ ID NO: 13 |
| *LINC02035* | GAAGCAAGCCAGAAAGCCTG (sense, NR_024618.1) | SEQ ID NO: 14 |
| | GTGGTGCAGAGCCATGAAAG (antisense) | SEQ ID NO: 15 |
| *LOC100287175* | GATCAGTCTCTCTGCGCCTC (sense, NR_149003.1) | SEQ ID NO: 16 |
| | GTGTCTCTTGGCCCTGTACC (antisense) | SEQ ID NO: 17 |
| *LMNTD2-AS1* | CAGGCACTCACCTACCTGAC (sense, NR_147607.1) | SEQ ID NO: 18 |
| | CCTGGAGCAGAGGGAATACTTG (antisense) | SEQ ID NO: 19 |
| *LOC100287808* | ACTGGGGAACTCAAGGCACAG (sense, NR_149004.1) | SEQ ID NO: 20 |
| | AGATTCGGCTCAAAGGAGGC (antisense) | SEQ ID NO: 21 |
| *LOC100130207* | GCACAACAGGACAAAGGAAGG (sense, NR_149025.1) | SEQ ID NO: 22 |
| | CAGATCGCCGTTTGCTTGG (antisense) | SEQ ID NO: 23 |
| *H19* | AAGAAATGGTGCTACCCAGC (sense, NR_002196.2) | SEQ ID NO: 24 |
| | GTGCAGTGGTTGTAAAGTGC (antisense) | SEQ ID NO: 25 |
| *OBSCN-AS1* | CTCCTCGATGCCGTACTTG (sense, NR_073154.1) | SEQ ID NO: 26 |
| | CCACGTTCCTATGTGAGGTG (antisense) | SEQ ID NO: 27 |
| *FOXD2-AS1* | CCTCCTACCAATTACACGCC (sense, NR_026878.1) | SEQ ID NO: 28 |
| | GTACACACAGGTCGCTATGG (antisense) | SEQ ID NO: 29 |
| *LOC101927811* | TTAAACGAGCCCTGGATCTG (sense, NR_110119.1) | SEQ ID NO: 30 |
| | CCCGAAACAGACTTCTGAAC (antisense) | SEQ ID NO: 31 |
| *ARHGAP5-AS1* | ATCGCTCGCCAACTACAGAC (sense, NR_027263.1) | SEQ ID NO: 32 |
| | TGGCTTCTCGCTCCACTTTC (antisense) | SEQ ID NO: 33 |
| *A2M-AS1* | GGGTAGCATAGTGCCCAAGG (sense, NR_137424.1) | SEQ ID NO: 34 |
| | TTTATTGGAGAGGGCCGCTG (antisense) | SEQ ID NO: 35 |
| *LOC100129034* | TTGCTGATGACGCAGAGGTC (sense, NR_027406.1) | SEQ ID NO: 36 |
| | GGACATTATTGCCGCCGTTC (antisense) | SEQ ID NO: 37 |
| *TSC22D1-AS1* | GAGAGCGAGCTTCGGAAAGG (sense, NR_038381.1) | SEQ ID NO: 38 |
| | TTCCTTTGAACGGCGTCGG (antisense) | SEQ ID NO: 39 |
| *LOC90246* | CTCCCAACCTCACCTGTGC (sense, NR_026954.1) | SEQ ID NO: 40 |
| | TAGCAGGGCTTTGGATGTGC (antisense) | SEQ ID NO: 41 |
| *SNAP25-AS1* | AGCACCCTAACGACAACAGTC (sense, NR_040710.1) | SEQ ID NO: 42 |
| | AGAACCCAGCTCCATTCATCC (antisense) | SEQ ID NO: 43 |
| *LIFR-AS1* | TTACAGGTGTCTGTGCGGTG (sense, NR_103554.1) | SEQ ID NO: 44 |
| | TCCAGTCAATCCTTGGCATCC (antisense) | SEQ ID NO: 45 |
| *MKLN1-AS* | ATCTCAGGGTTTCCCACGTC (sense, NR_125364.1) | SEQ ID NO: 46 |
| | ACTGTCCAGGCTTTCAGGAG (antisense) | SEQ ID NO: 47 |
| *LINC02015* | ACAGCCGGGAAGAAAATTGT (sense, NR_110826.1) | SEQ ID NO: 48 |
| | CCAGAAGTTCATGGCAGCAG (antisense) | SEQ ID NO: 49 |
| *SOX9* | Cat. No- P232240 V (RNA accession: NM_000346.3) | -. |
| | https://www.bioneer.co.kr/sirna-customorder.html | |
| *COL2A1* | Cat. No- P298511 V (RNA accession: NM_001844.4)https://www.bioneer.co.kr/sirna-customorder.html | -. |
| β-ACTIN | GTCCTCTCCCAAGTCCACACA (sense, NM_001101.3) | SEQ ID NO: 50 |
| | GGGCACGAAGGCTCATCATTC (antisense) | SEQ ID NO: 51 |

### Western blot analysis

TC28a2 cells or MSCs were lysed in PROPREP^{™} protein extraction solution (iNtRON Biotechnology, Seongnam, South Korea). Protein concentration was determined using the Bio Rad Protein Assay (Bio-Rad Laboratories, Inc., Hercules, CA, USA). Approximately 20-30 µg of protein was analyzed by 10% SDS-polyacrylamide gel electrophoresis (SDS-PAGE; Sigma-Aldrich). Separated proteins were transferred to the membrane and blocked with 5% skim milk (BD, Sparks, MD, USA) for 1 h at room temperature and the membrane was probed with the following antibodies [COX2 (1:1000; BD Biosciences, San Jose, CA, USA), IHH (1:1000; Santa Cruz Biotechnology), RUNX2 (1:1000; Santa Cruz Biotechnology), COL10A1 (1: 1000; Abcam, Cambridge, UK), COL2A1 (1:1000; Santa Cruz Biotechnology), ACAN (1:1000; Santa Cruz Biotechnology), MMP13 (1:1000; Cell Signaling Technology, Danvers, MA, USA), ubiquitin (1:1000; Santa Cruz Biotechnology), SOX9 (1:1000; Santa Cruz Biotechnology), FLAG (1:1000; Sigma-Aldrich), and HSP90 (1:5000; Santa Cruz Biotechnology)] and incubated overnight. The same was done with an antibody against β-actin (1:5000; Santa Cruz Biotechnology), which serves as a loading control.

### Human OCN pGreenZeo Differentiation Reporter Assay

To establish TC28a2 cells stably expressing copGFP with an active OCN promoter/enhancer, a lentiviral plasmid vector for the humanOCN pGreenZeo differentiation reporter was purchased (System Biosciences, LLC; SBI SR1003PA-1). To obtain lentiviral particles with the human OCN pGreenZeo differentiation reporter, HEK293T cells were inoculated into T75 flasks at a density of 5 x 106 cells per plate. Adherent cells were transfected with lentiviral plasmids for human OCN pGreenZeo differentiation reporters with psPAX2 (http://www.addgene.org/12260/) and pMD2.G (http://www.addgene.org/12259/) using CalFectin mammalian cell transfection reagent (SignaGen Laboratories, Frederick, MD, USA). The medium was replaced approximately 6 hours after transfection. Transfected cells were maintained for 2 days, and supernatants were harvested and stored at -70°C. To monitor GFP activity in TC28a2 cells transfected with the OCN pGreenZeo differentiation reporter lentivirus, cells were inoculated into 6-well plates at a density of 1 x 10⁵ cells under hypertrophic differentiation or inflammatory conditions. Five days after induction of hypertrophic differentiation or inflammation, copGFP signal was observed using fluorescence microscopy. For quantitative analysis, cells were treated with passive lysis buffer (Promega, Madison, WI, USA) and incubated at 4°C for 5 min. The whole lysates were transferred to microcentrifuge tubes and centrifuged to obtain supernatants. After determining the total protein concentration of each lysate sample, fluorescence was measured at 360 nm/465 nm (excitation/emission) using a fluorescence plate reader.

### RNA sequencing and data analysis

To prepare samples for RNA sequencing, total RNA was extracted from TC28a2 cells treated with growth medium (n = 2) or hypertrophic medium (n = 3) on day 5 of differentiation. RNA was extracted using TRIzol (Invitrogen) according to the protocol provided by the manufacturer. The overall quality of the extracted total RNA was verified using spectrophotometry. Raw reads were read on the sequencer prior to analysis to remove low-quality and adapter sequences, and the processed reads were aligned to Homosapiens using HISAT2 v2.1.0. The reference genome sequence and annotation data for Homo sapiens (hg19) were downloaded from NCBI, and transcriptome assemblies of known transcripts were processed using StringTie v2.1.

Based on these results, transcriptome and gene expression levels were calculated as fragments per kilobase of exon per million fragments mapped (FPKM) values or read counts per sample. The expression profiles were used to perform further analysis, such as determining the number of differentially expressed genes (DEGs). StringTie was used to measure the relative abundance of genes in the read counts. Genes with more than one read count value in the sample were excluded, and the filtered data were log2 transformed and RLE normalized. Statistical significance of differential expression data was determined using DESeq2 nbinomWaldTest and fold change, where the null hypothesis is that no difference exists between groups. False discovery rate (FDR) was controlled by adjusting the p-value using the Benjamini-Hochberg algorithm, and for DEG sets, hierarchical clustering analysis was performed using complete linkage and Euclidean distance as measures of similarity. Gene ontology analysis was performed for DEGs using g:Profiler and KEGG pathway analysis was performed based on the KEGG pathway (https://www.genome.jp/kegg/) database. Multidimensional scaling (MDS) method was used to visualize the similarity between samples, and Euclidean distance was applied as a measure of dissimilarity. Hierarchical clustering analysis was performed using complete linkage and Euclidean distance as similarity measures to display the expression patterns of differentially expressed transcripts satisfying fold change ≥ 2 and raw P < 0.05. All data analysis and visualization of differentially expressed genes were performed using R (version 3.6.1; www.r-project.org), and RNA sequencing data are available in the GEO database under accession number GSE199847

### RNAi

All siRNAs used in this invention were purchased from Bioneer and information about the siRNAs used is shown in Table 2 below. TC28a2 cells or MSCs were plated to obtain 70-80% confluent growth in 6-well plates and transfected with 100 nM of each siRNA using Lipofectamine 2000 (Invitrogen). Six hours after transfection, the medium was replaced with fresh medium. The next day, cells were detached from the plates, counted, and re-inoculated at the appropriate cell density for each experiment.

**[Table 2]**

| siRNAs | Dose (nM) | Target sequence (5'→3') |
|---|---|---|
| Negative control | 100 | Sense: CCUACGCCACCAAUUUCGU (SEQ ID NO: 52) |
| | | Antisense: ACGAAAUUGGUGGCGUAGG (SEQ ID NO: 53) |
| *LINC02035* | 100 | Sense: CAGGAUUGAGGAAUCUGUA (SEQ ID NO: 54) |
| | | Antisense: UACAGAUUCCUCAAUCCUG (SEQ ID NO: 55) |
| *LOC100130207* | 100 | Sense: CAGAACUGCCAGCAGCAGACUA (SEQ ID NO: 56) |
| | | Antisense: UAGUCUGCUGGCAGUUCUG (SEQ ID NO: 57) |
| *LINC02593* | 100 | Sense: GAUUACAGAGUCAUUCAUUU (SEQ ID NO: 58) |
| | | Antisense: AAUGAAUGACUCUGUAAUC (SEQ ID NO: 59) |
| *LOC100287808* | 100 | Sense: CAAUUACCUCCAGCAUAU (SEQ ID NO: 60) |
| | | Antisense: UAUAUGCUGGAGGUAAUUG (SEQ ID NO: 61) |
| *LMNTD2-AS1* | 100 | Sense: CUGACUUGCUGAAACUACA (SEQ ID NO: 62) |
| | | Antisense: UGUAGUUUCAGCAAGUCAG (SEQ ID NO: 63) |
| *HCG4B* | 100 | Sense: CUGCAAAGAAGGAACACUA (SEQ ID NO: 64) |
| | | Antisense: UAGUGUUCCUUCUUCUUUGCAG (SEQ ID NO: 65) |
| *LOC100287175* | 100 | Sense: GCAGCCUCCUCUUGUUCCU (SEQ ID NO: 66) |
| | | Antisense: AGGAACAAGAGGAGGAGGCUGC (SEQ ID NO: 67) |
| *IGFL2-AS1* | 100 | Sense: GACUCUCUUCAUAUGGACU (SEQ ID NO: 68) |
| | | Antisense: AGUCCAUAUGAAGAGAGAGUC (SEQ ID NO: 69) |

### RNA-IP

RNA-IP was performed to identify potential interactions between the RUNX2 protein and the newly discovered in the present invention. TC28a2 cells (1 x 10⁷ ) transfected with the pcDNA3.1-RUNX2 vector were used for each experiment. RUNX2-bound RNA-IP was performed using the RiboCluster Profiler RIP assay kit protocol (MBL International Corporation, Woburn, MA, USA). Cells were lysed using manufacturer's lysis buffer supplemented with dithiothreitol (DTT; Sigma), and lysates were precleared by adding protein G and agarose beads (Thermo Fisher Scientific) in manufacturer's DTT-supplemented wash buffer. The precleared lysate was transferred to prepared tubes containing RUNX2 antibody (Santa Cruz Biotechnology) or IgG-immobilized beads. After incubation overnight, the RUNX2 antibody or IgG-immobilized protein G agarose bead RNA/protein complexes were separated to extract protein and PUM1-bound RNA. Eluted RNA was analyzed by qPCR. RUNX2 antibody-bound RNA was isolated and purified using the kit according to the manufacturer's instructions.

### Enzyme-linked immunosorbent assay (ELISA) for RUNX2 activity

The activity measurement of RUNX2 was performed using an ELISA kit for human runt-related transcription factor 2 (Cloud-Clone Corp., Katy, TX, USA) according to the manufacturer's instructions. TC28a2 cells were lysed before the assay according to the manufacturer's instructions, and the samples used or the standards provided by the manufacturer were transferred to plates pre-coated with antibodies, and the samples were incubated at 37°C for 1 h. After aspiration of each sample, the prepared detection reagent A was added, and the plates were incubated at 37°C for 1 h. After washing, the detection reagent B was added. After washing, Detection Reagent B was added and the plate was incubated at 37°C for 30 minutes. The solution on the plate was aspirated and washed with the manufacturer's wash buffer, the substrate solution was added to the plate, and the plate was incubated again at 37°C for 20 minutes. After the addition of the stop solution, the plates were read using a spectrophotometer microplate reader at 450 nm. All samples and standards were tested in triplicate.

### LINC02035 and LOC100130207, lncRNAs, in situ hybridization

Cell localization and in situ photography of LINC02035 and LOC1001303207 were performed using the RNAscope 2.5 HD Assay-RED kit (Advanced Cell Diagnostics, Hayward, CA, USA) according to the manufacturer's instructions. For sample preparation, articular cartilage tissue obtained from osteoarthritis patients undergoing total knee replacement was shaved from the articular surface under sterile conditions, divided into intact and damaged parts, and minced thoroughly. Chondrocytes were then primary isolated from either intact or damaged cartilage, respectively, and cells were cultured in 4-well glass chamber slides (Nalge Nunc International, Rochester, NY, USA) in a 5% CO₂ incubator at 37°C overnight. The next day, cells were immediately used for RNA in situ hybridization analysis and standard positive control (Hs-PPIB, ACD-313901) and negative control (DapB, ACD-310043) probes were used to validate the results. Probes for in situ hybridization of LINC02035 (Hs-LINC02035, ACD 519 1116341-C1) and LOC100130207 (Hs-LOC100130207-C1, ACD-1116351-C1. Purchased from Advanced Cell Diagnostics) were also prepared, and the sequence information for each probe is as follows (Hs-LINC02035-C1 [Cat. No. 1116341; accession no. NR_024618.1; No. of pairs 20; target region 1451-2476] and Hs-LOC100130207-C1 [C Cat. No. 1116351; accession no. NR_149025.1; No. of pairs 20; Target region 645-1765]). All experimental procedures for RNA in situ hybridization were performed strictly according to the manufacturer's instructions.Cells seeded in 4-well glass chamber slides were fixed in 10% neutral buffered formalin, and the slides were treated with peroxidase blocking solution for 15 min at room temperature. Afterward, each slide was treated with the recovery solution for 15 minutes at 100°C. The hybridization step was performed in an oven provided by Advanced Cell Diagnostics at 40°C for 2 hours. After the washing step, the samples were incubated with the signal amplification solution as follows: Amp1 at 40°C for 30 minutes, Amp2 at 40°C for 15 minutes, Amp3 at 40°C for 30 minutes, Amp4 at 40°C for 15 minutes, Amp5 at room temperature for 30 minutes, and Amp6 at room temperature for 15 minutes. The samples were then incubated with Fast Red A and B solutions to visualize each target lncRNA-positive spot and incubated with hematoxylin for control staining. The results were observed using a standard microscope.

### RNA Subcellular Fractionation

Fractionation of cytoplasmic and nuclear RNA from TC28a2 cells was performed using the RNA Subcellular Isolation Kit (Active Motif, Inc., Carlsbad, CA, USA) according to the manufacturer's instructions. Cytoplasmic and nuclear RNA isolated from TC28a2 cells were reverse transcribed, and the expression levels of each lncRNA discovered in the present invention were evaluated by qPCR. Data normalization was performed with respect to total cellular RNA [% of input = 100 x [2^{(Ct total RNA- Ct RNA fraction)}] to obtain the percentage of cytoplasmic and nuclear RNA.

### IP

Lysates of TC28a2 cells transfected with pcDNA3.1-RUXN2 or siRNA targeting LINC02035 or LOC100130207 were prepared, and the prepared lysates were incubated with protein A/G agarose beads (Santa Cruz Biotechnology) and antibody against RUNX2 (Santa Cruz Biotechnology). The lysate and RUNX2 antibody-bound beads were centrifuged and washed three times with lysis buffer. The complexes were separated from the beads using 2x SDS sample dye and subjected to Western blotting. All membranes were incubated with antibodies against RUNX2 (Santa Cruz Biotechnology), ubiquitin (Santa Cruz Biotechnology), or HSP90 (Santa Cruz Biotechnology) for 12 hours, followed by incubation with HRP-conjugated secondary antibodies for 1 hour.

### Safranin O, H&E, and IHC staining

Mesenchymal stem cells (hMSCs) were transfected with siRNAs (siLINC02035 or siLOC100130207), respectively, and three 3D masses (3 x 10⁵ cells/1 mass) per group were used to induce chondrogenic differentiation [(DMEM-HG supplemented with 1% antibiotic-antimycotic solution, 1% Insulin Transferrin Selenium-A (ITS; Invitrogen), 50 mg/mL ascorbic acid (Invitrogen), and 10 ng/mL TGF-b3 (R&D Systems)] for 14 days, cartilage pellets were mixed with fibrin gel (20 ul/3 mass), and masses were implanted and sutured through an approximately 5-mm incision in the lower dorsal skin of anesthetized nude mice. The resulting tissue was isolated for histologic analysis 7 days after implantation. The isolated tissues were fixed in formalin for 24 hours and paraffin embedded. Paraffin blocks were cut into 6 um thick sections and mounted on slides, and the slides were stained with safranin-O (1% in 1% acetic acid)/fast green (0.02% in D.W.), H&E staining. For immunohistochemistry (IHC), antigen retrieval was induced with RUNX2 (Santa Cruz, sc-390351), IgG (BD Bioscience, 555746), and overnight incubation at 4°C. HRP polymer mouse/rabbit antibody (secondary antibody) was incubated for 1 h at room temperature, followed by final staining with AEC substrate kit (abcam), and observed under a microscope.

### Statistical analysis

All experiments were performed in triplicate using samples from at least three donors, and Student's t-test was used to evaluate differences between two groups. The statistical significance of differences between three or more groups was calculated using one-way analysis of variance (ANOVA) and post hoc Bonferroni correction, and each data is presented as mean ± standard deviation. For all tests, P < 0.05 was considered statistically significant, and interaction strengths were estimated using RPISeq (http://pridb.gdcb.iastate.edu/RPISeq/index.html).

Exclusion criteria for cartilage tissue in osteoarthritis patients undergoing total knee arthroplasty were rheumatoid arthritis, inflammatory arthritis, autoimmune disease, ankylosing spondylitis, cancer, and a history of long-term corticosteroid treatment.

All statistical analyses in the present invention were performed by analysis of variance (ANOVA) or Student's t-test using GraphPad Prism6 software, and each data was calculated as mean ± standard deviation. A p-value of less than 0.05 was considered statistically significant.

### Experimental results

### 1.Relationship between RUNX2 and chondrocyte hypertrophy

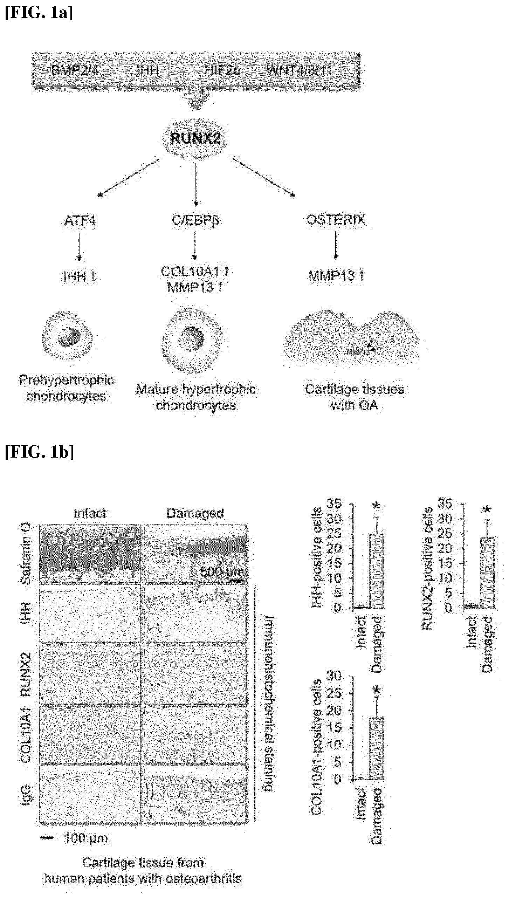

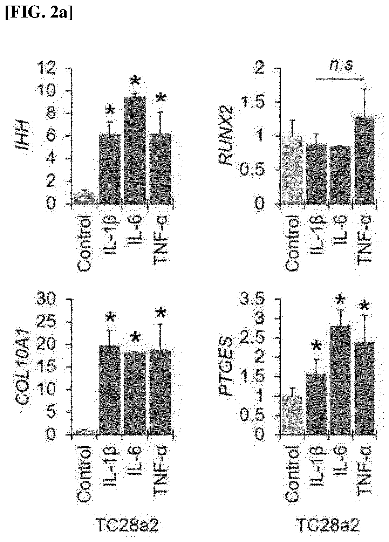

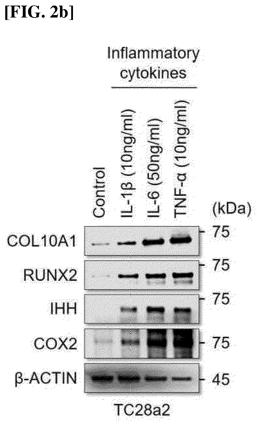

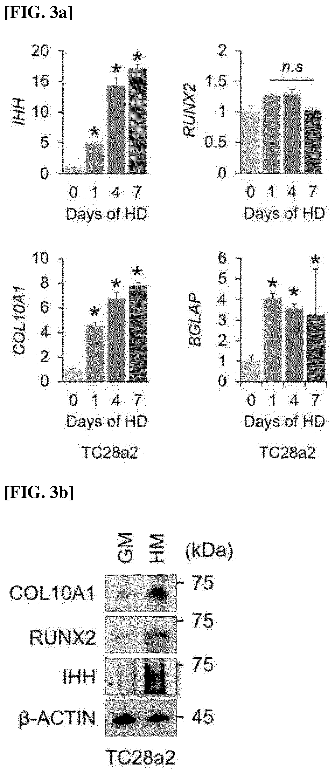

The RUNX2 transcription factor is a central regulator of hypertrophic changes in chondrocytes, as shown in Figure 1a, and is known to be involved in the degradation of the cartilage matrix. To determine the regulatory role of RUNX2 in chondrocyte hypertrophic changes and cartilage matrix degradation, we first performed immunohistochemistry (IHC) to detect hypertrophy markers, including RUNX2, in cartilage tissues obtained from OA patients, and found that the chondrocyte hypertrophy markers IHH, RUNX2, and COL10A1 were highly detected in damaged cartilage tissues, whereas they were barely detected in normal cartilage tissues (see Figure 1b). To observe changes in chondrocyte hypertrophy in an in vitro setting, we used TC28a2 cells, a normal chondrocyte cell line, and to mimic the osteoarthritis environment in vitro, we used the inflammatory cytokines interleukin-1β (IL-1β), interleukin-6 (IL-6), and tumor necrosis factor-α (TNF-α). We found that inflammatory cytokines upregulated the mRNA and protein levels of PTGES (inflammation marker COX2) and the hypertrophy markers IHH and COL10A1, and interestingly, the amount of RUNX2 protein was upregulated by inflammatory cytokines, but the mRNA level was unchanged (see Figure 2a and Figure 2b). We used hypertrophy medium (HM) to create an *in vitro* environment specific for chondrocyte hypertrophy and similarly found that hypertrophy medium upregulated both mRNA and protein levels of IHH and COL10A1, markers of chondrocyte hypertrophy, in TC28a2 cells, whereas hypertrophy medium improved protein levels of RUNX2 but did not change mRNA levels (see Figure 3a and Figure 3b).

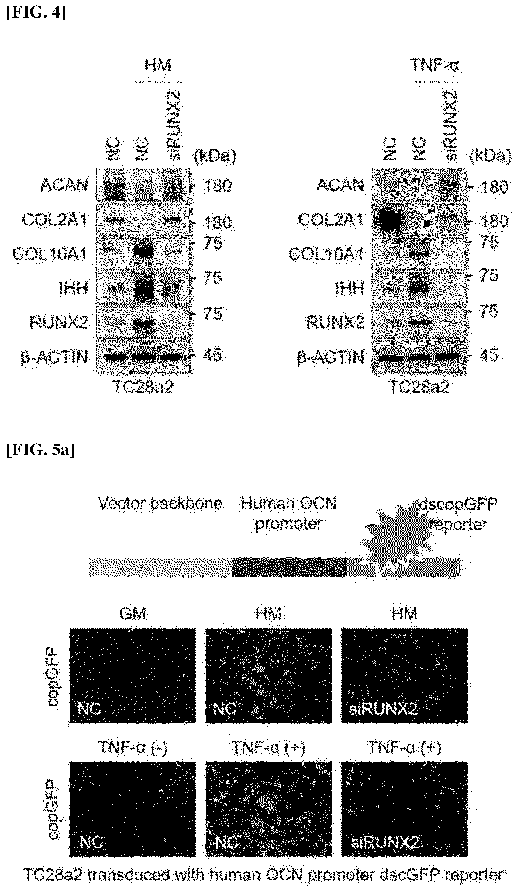

To further clarify whether RUNX2 is functionally associated with hypertrophic changes in chondrocytes, the inventors performed siRNA-mediated knockdown (KD) experiments in hypertrophic and inflammatory settings and found that knockdown of RUNX2 protected chondrocytes from the loss of cartilage matrix proteins COL2A1 and ACAN induced by hypertrophic medium or TNF-α treatment. Furthermore, knockdown of RUNX2 suppressed the upregulation of IHH and COL10A1 markers (see Figure 4).

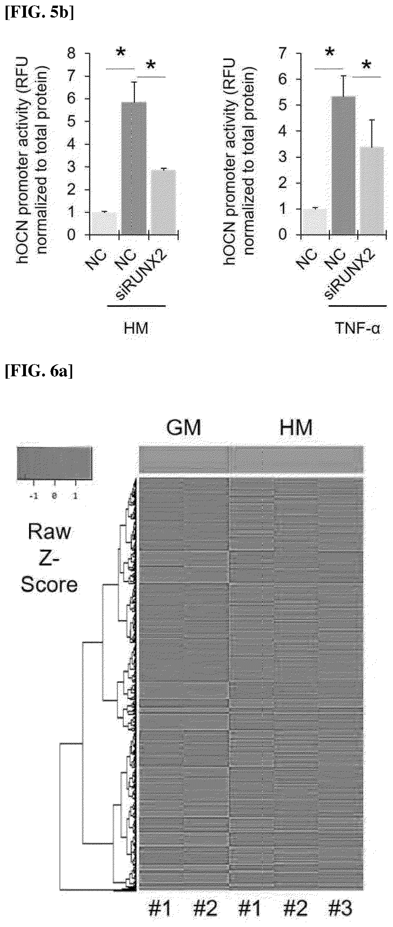

As osteocalcin (OCN), a protein produced by osteoblasts in bone, is known to be involved in matrix calcification of cartilage tissue, we performed a human OCN pGreenZeo (copGFP) differentiation reporter assay to determine whether OCN activity in chondrocytes is regulated by hypertrophic medium or TNF-α treatment. TC28a2 cells were transfected with a lentiviral vector encoding the OCN promoter and copGFP, selected with xeomycin, and used for the reporter assay. The results showed that a strong copGFP signal was detected in TC28a2 cells treated with hypertrophic medium or TNF-α, and the increased copGFP signal was significantly reduced by siRNA-mediated KD of RUNX2 (see Figure 5a and Figure 5b). Taken together, the above results once again confirm that RUNX2 is a master regulator of chondrocyte hypertrophic changes.

### 2. interaction between LncRNA and RUNX2 protein

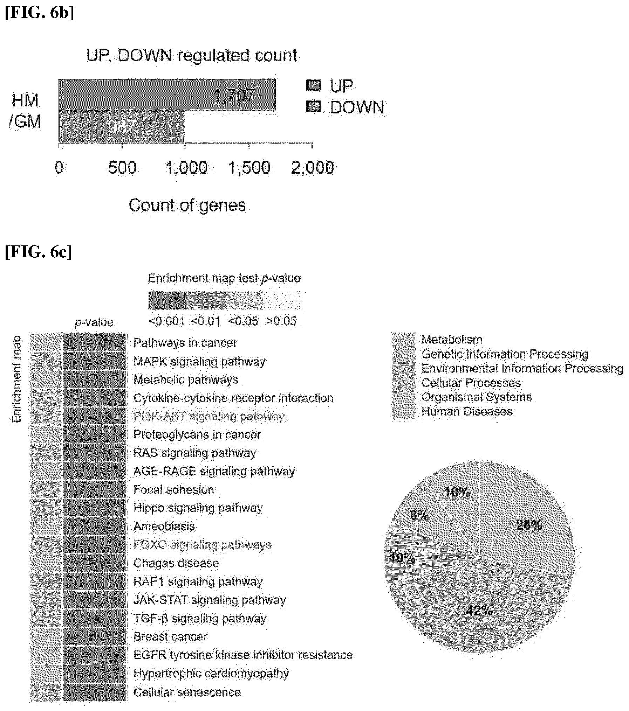

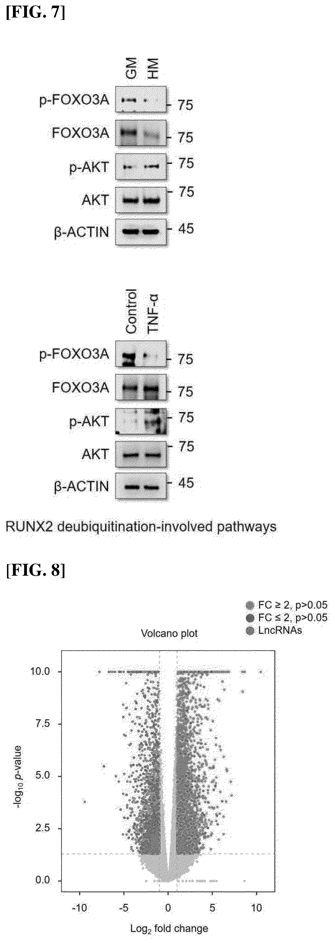

To identify the molecular mechanisms involved in chondrocyte hypertrophy, RNA sequencing analysis was performed using RNA extracted from TC28a2 cells grown in hypertrophic medium. Heatmaps were used to display the differential transcriptome between TC28a2 cells grown in growth medium or hypertrophic medium (see Figure 6a). A total of 1,707 transcripts were found to be up-regulated (log2 [fold-change] > 2, FDR < 0.05) and 987 transcripts were found to be down-regulated (log2 [fold-change] < -2, FDR < 0.05) in TC28a2 cells grown in hypertrophic differentiation medium (Figure 6b). KEGG pathway analysis identified that pathways related to environmental information processing and metabolism accounted for a significant proportion of the differentially regulated cell signaling pathways (Figure 6c). During the KEGG pathway analysis, we focused on changes in the PI3K-AKT and FOXO signaling pathways because they are involved in the stability of the RUNX2 protein. We found that AKT phosphorylation increased in TC28a2 cells treated with hypertrophic medium or TNFα, while FOXO3A phosphorylation decreased (see Figure 7). These results imply that RUNX2 may be regulated at the post-translational level during hypertrophic differentiation of chondrocytes.

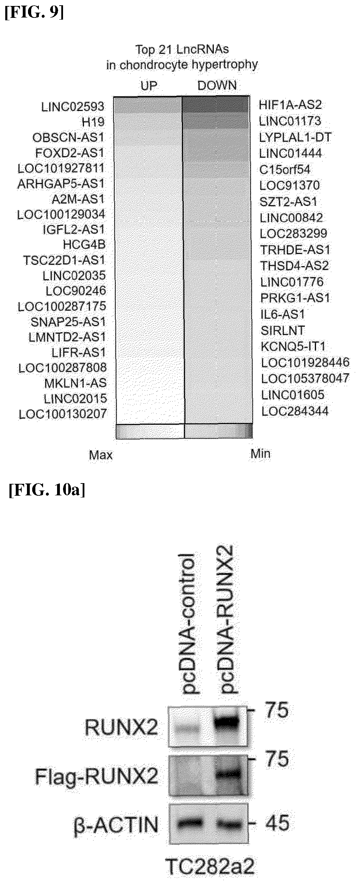

RNA sequencing analysis was performed by screening 2,181 protein-coding genes and 223 lncRNAs among the differentially expressed transcripts, and referring to Figure 8, it can be seen that there is a difference in the expression of lncRNAs and protein-coding mRNAs between the control and acromegaly groups. Here, it is understood that lncRNAs are known to regulate the activity of target proteins by physically binding to proteins, DNA or RNA. Since RUNX2 was shown to be regulated at the post-translational level as confirmed by the experimental results above, and differential expression of lncRNAs was observed in the RNA sequencing results, the inventors sought to identify specific lncRNAs involved in the stabilization of the RUNX2 protein among the numerous lncRNAs. The heatmap of 21 differentially expressed lncRNAs that were upregulated more than 4-fold or had the largest decrease in expression level during hypertrophic differentiation is shown in Figure 9. A computer algorithm (RNA-Protein Interaction Prediction; RPISeq) was then performed to predict which of the upregulated lncRNAs might interact with RUNX2. A value of 0.5 or higher was interpreted as a positive binding potential between the analyzed RNA and protein (http://pridb.gdcb.iastate.edu/RPISeq/about.php#basic). However, most of the analyzed lncRNAs were found to be positive, as shown in Table 3 below.

**[Table 3]**

| Rank | LncRNA name | RF classifier | SVM classifier |
|---|---|---|---|
| 1 | SNAP25-AS1 | 0.95 | 0.99 |
| 2 | ARHGAP5-AS1 | 0.95 | 0.95 |
| 3 | LIFR-AS1 | 0.9 | 0.99 |
| 4 | LINC02035 | 0.9 | 0.98 |
| 5 | MKLN1-AS | 0.9 | 0.97 |
| 6 | HCG4B | 0.95 | 0.9 |
| 7 | LOC 100130207 | 0.9 | 0.95 |
| 8 | LINC02015 | 0.9 | 0.94 |
| 9 | LOC101927811 | 0.85 | 0.96 |
| 10 | A2M-AS1 | 0.85 | 0.93 |
| 11 | LOC90246 | 0.85 | 0.92 |
| 12 | TSC22D1-AS1 | 0.8 | 0.96 |
| 13 | IGFL2-AS1 | 0.85 | 0.9 |
| 14 | LOC100287808 | 0.85 | 0.77 |
| 15 | OBSCN-AS1 | 0.85 | 0.69 |
| 16 | LOC100129034 | 0.8 | 0.68 |
| 17 | FOXD2-AS1 | 0.95 | 0.48 |
| 18 | LINC02593 | 0.8 | 0.47 |
| 19 | LMNTD2-AS1 | 0.8 | 0.45 |
| 20 | H19 | 0.7 | 0.34 |
| 21 | LOC100287175 | 0.55 | 0.3 |

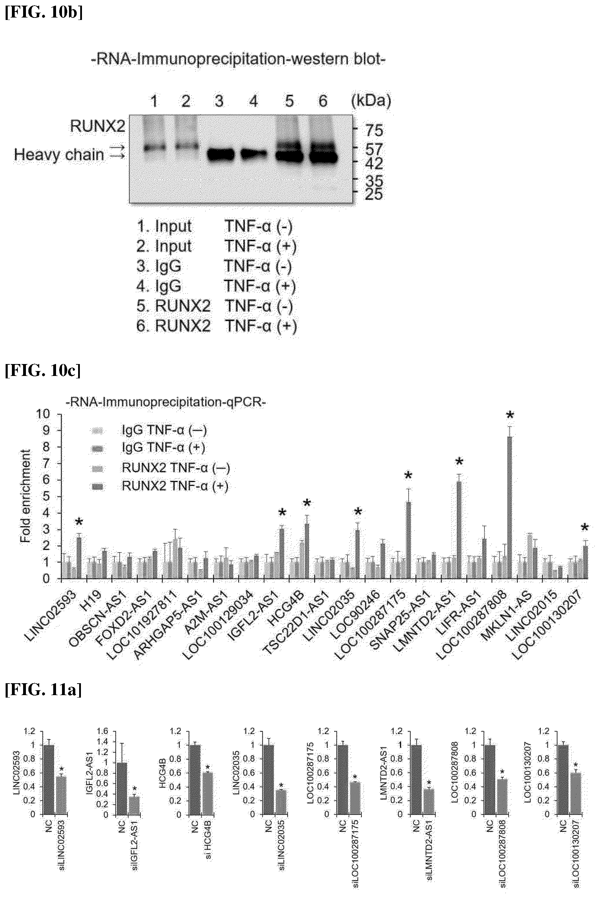

Accordingly, RNA-immunoprecipitation (RNA-IP) was performed to identify lncRNAs that can bind to RUNX2, and vectors encoding RUNX2 were transfected into TC28a2 cells, and the IP results for RUNX2 were confirmed (see Figure 10a and Figure 10b). As a result, a total of eight lncRNAs were found to accumulate from RUNX2 protein during inflammatory response in TC28a2 cells (see Figure 10c).

### 3. identify novel LncRNAs that interact with RUNX2 in chondrocytes

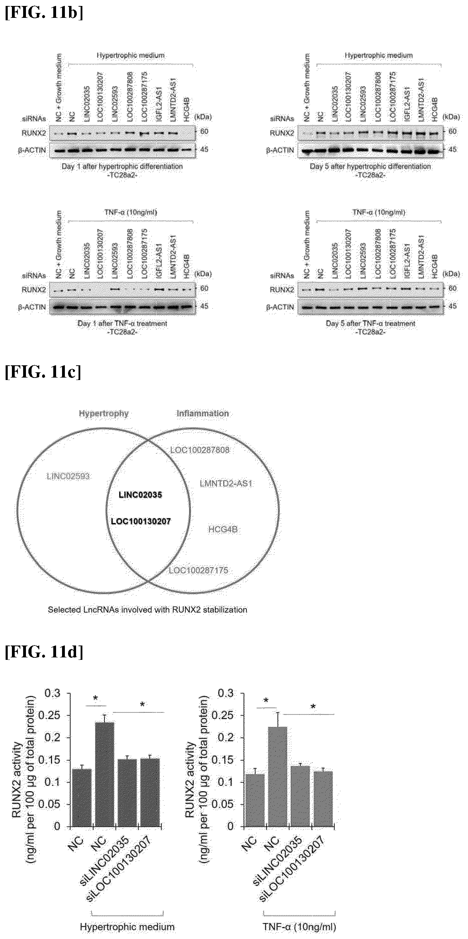

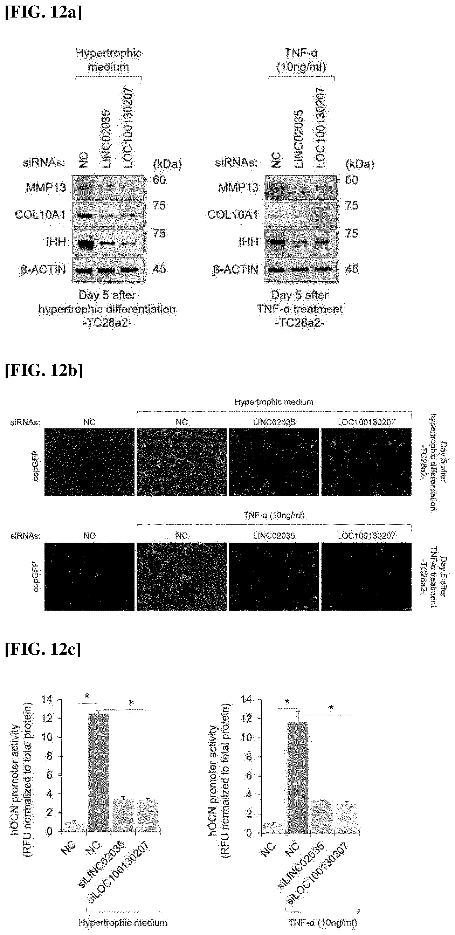

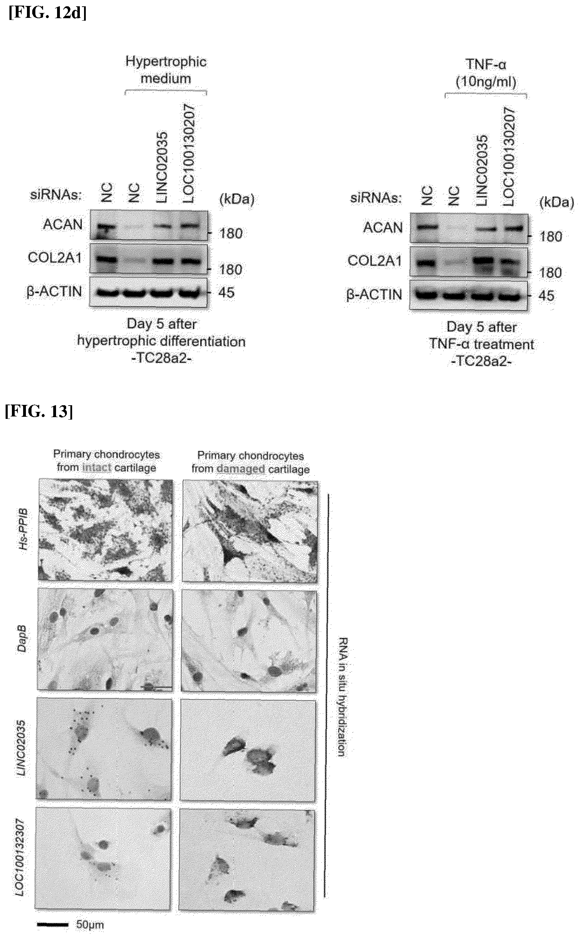

To determine whether the RUNX2-binding lncRNAs could functionally contribute to the stability of the RUNX2 protein, siRNA-mediated knockdown experiments were performed for each lncRNA. The KD efficiency of each siRNA used was confirmed by real-time quantitative polymerase chain reaction (qPCR) (see Figure 11a). TC28a2 cells were treated with hypertrophic medium or TNF-α, and the quantitative changes in RUNX2 protein when each lncRNA was knocked down were determined by western blotting, and the results are shown in Figure 11b. Notably, TC28a2 cells transfected with siRNAs against LINC02035 or LOC100130207 did not show an increase in RUNX2 protein levels under either hypertrophic or inflammatory conditions (see Figure 11c). For further analysis, we measured RUNX2 activity in TC28a2 cells transfected with siRNAs against LINC02035 or LOC100130207 and found that RUNX2 activity was significantly reduced by knockdown of these two lncRNAs (see Figure 11d). These results suggest that LINC02035 and LOC100130207 functionally interact with RUNX2 in chondrocytes. Next, we sought to determine whether LINC02035 or LOC100130207 was involved in the increase of chondrocyte hypertrophy markers and the degradation of cartilage matrix proteins. Western blotting confirmed that knockdown of these two lncRNAs suppressed the upregulation of hypertrophy markers such as IHH, COLA10A1 and MMP13 under hypertrophic or inflammatory conditions (see Figure 12a). Furthermore, the promoter activity of OCN was not increased by knockdown of these two lncRNAs in each condition (see Figure 12b and Figure 12c). Furthermore, knockdown of these two lncRNAs prevented chondrocyte hypertrophy and the decrease of cartilage matrix proteins COL2A1 and ACAN, which are destroyed by inflammatory responses (see Figure 12d). These results confirm that knockdown of LINC02035 and LOC100130207, among other lncRNAs, can protect chondrocytes from hypertrophy and inflammatory response.

### 4. confirm the effect of LINC02035 and LOC100130207 (1) - stabilizing the RUNX2 protein

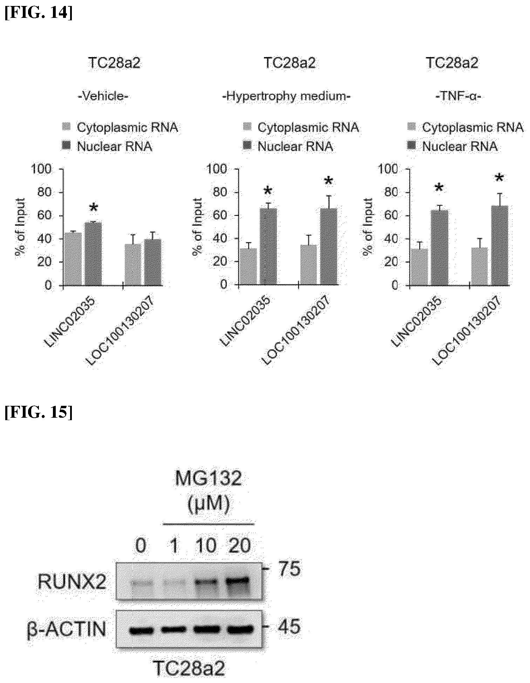

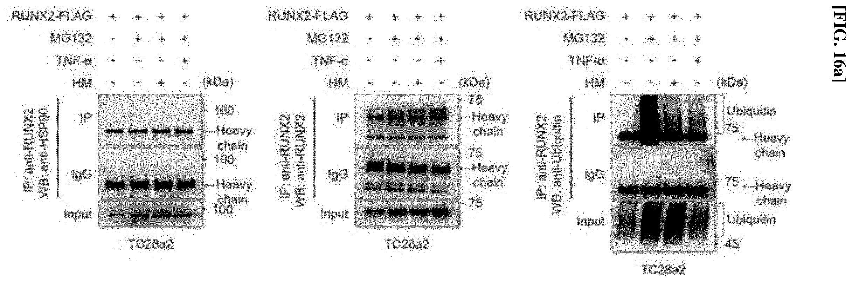

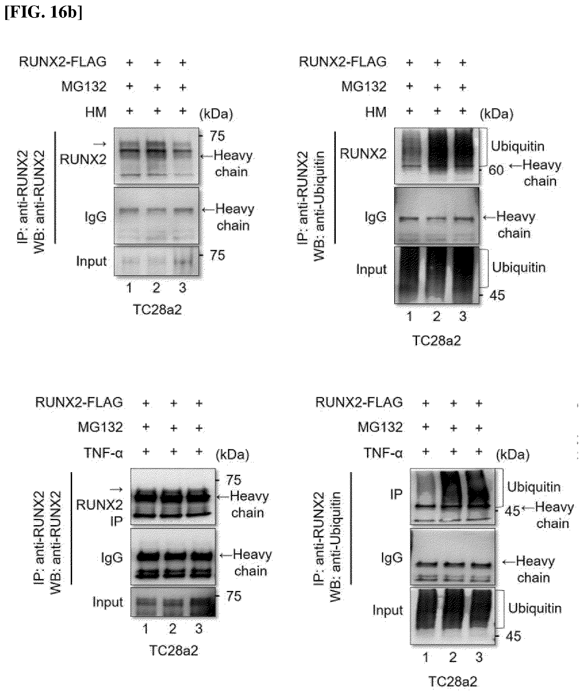

To validate the molecular mechanism of the newly discovered lncRNAs, LINC02035 and LOC100130207, the inventors performed in situ analysis using primary chondrocytes derived from patients. As a result, we found that both LINC02035 and LOC100130207 were highly expressed in chondrocytes isolated from knee cartilage of osteoarthritis (OA) patients (see Figure 13). Therefore, we performed subcellular localization analysis of these two lncRNAs in TC28a2 cells to elucidate the sites of action of these two lncRNAs. As a result, the leftmost graph in Figure 14 shows that LINC02035 and LOC100130207 were evenly distributed in the nucleus and cytoplasm of normal chondrocytes, while the middle and right graphs in Figure 14 show that both lncRNAs were preferentially distributed in the nucleus and to a lesser extent in the cytoplasm after TC28a2 cells were treated with hypertrophic medium or TNF-α. To determine whether RUNX2 is regulated at the post-translational level during chondrocyte hypertrophic changes, TC28a2 cells were treated with the proteasome inhibitor MG132. The results confirmed that the protein level of RUNX2 was dose-dependently increased by MG132 treatment (see Figure 15), suggesting that the upregulation of RUNX2 in osteoarthritic or hypertrophic chondrocytes occurs at the post-translational level. To investigate whether the two lncRNAs are involved in the stabilization of RUNX2 protein and inflammatory response during hypertrophic differentiation of chondrocytes, we performed IP to determine the ubiquitination of RUNX2 protein. We first investigated how ubiquitination of RUNX2 protein is regulated during hypertrophic differentiation or inflammatory response of chondrocytes, and found that ubiquitination of RUNX2 was reduced by hypertrophic medium or TNF-α treatment (Figure 16a). However, ubiquitination of RUNX2 protein is clearly increased in TC28a2 cells transfected with siRNA targeting LINC02035 or LOC100130207, even though the cells were treated with hypertrophic medium or TNF-α (see Figure 16b). These results suggest that both the upregulated lncRNAs and the inflammatory response of chondrocytes during hypertrophic differentiation contribute to the stabilization of RUNX2 protein.

### 5. confirmation of the effects of LINC02035 and LOC100130207 (2) - inhibition of chondrocyte hypertrophy

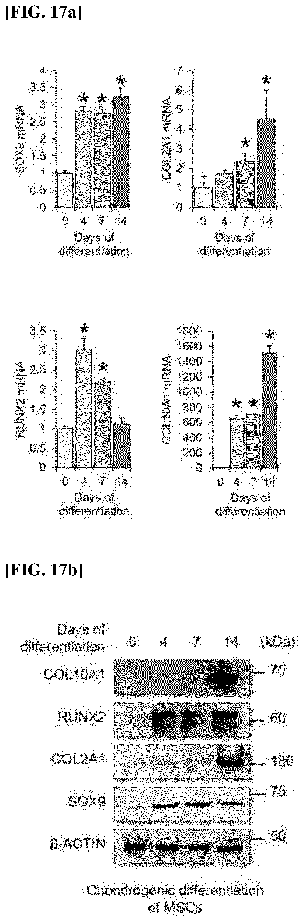

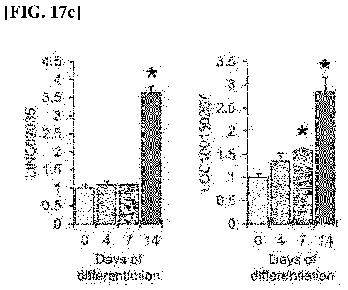

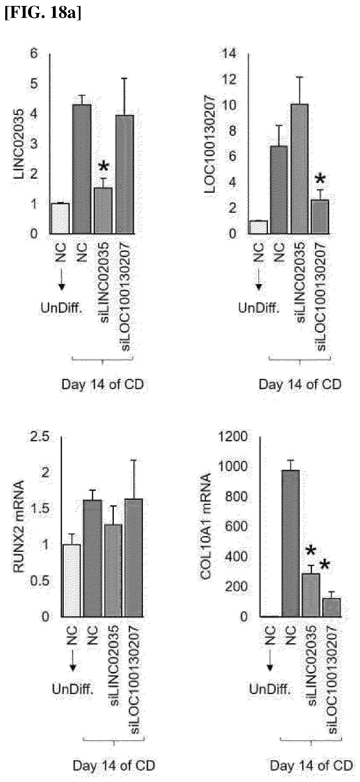

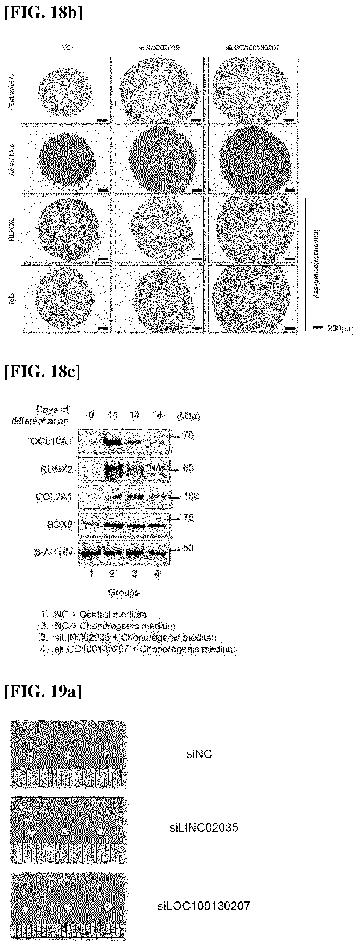

It is already known that mesenchymal stem cells (MSCs) have great potential to regenerate damaged cartilage tissue, but their clinical application has major limitations. In particular, chondrocyte hypertrophy is an essential process for endochondral ossification, which can be problematic for the regeneration of articular cartilage where chondrocytes maintain their stable properties. Therefore, successful cartilage regeneration can be achieved by using chondrogenic MSCs that lack the hypertrophic phenotype. We first checked the expression levels of mRNA and protein for chondrogenic and hypertrophic markers and found that the mRNA and protein levels of chondrogenic (SOX9 and COL2A1) and hypertrophic markers (COL10A1) increased significantly during differentiation, but in RUNX2, the protein levels were continuously upregulated during differentiation, while the mRNA levels tended to decrease back to basal levels in the later stages of differentiation (see Figure 17a and Figure 17b). Interestingly, we found that the expression levels of LINC02035 and LOC100130207 were only upregulated in the later stages of differentiation, regardless of the mRNA pattern or protein levels of RUNX2 (see FIG. 17c). To evaluate whether the two lncRNAs could affect the hypertrophic phenotype in chondrogenic differentiation of MSCs, we knocked down LINC02035 and LOC100130207 using siRNA, and identified the associated phenotypes. We found that both lncRNAs were well reduced by their respective siRNAs, and the mRNA levels of COL10A1 were also reduced, while the mRNA levels of RUNX2 were unchanged (see Figure 18a). Staining with safranin O and alcian blue confirmed that knockdown of both lncRNAs had no significant effect on the synthesis of glycosaminoglycan, but the protein of RUNX2 was barely expressed in the siRNA-transfected group (see Figure 18b). Interestingly, the size of the chondrogenic mass transfected with each siRNA was larger than that of the control group. Western blot results confirmed that knockdown of LINC02035 or LOC100130207 suppressed the upregulation of hypertrophy markers RUNX2 and COL10A1, while the protein levels of chondrogenic markers SOX9 and COL2A1 were almost unchanged in the siRNA-transfected groups (Figure 18c). These results suggest that LINC02035 and LOC100130207 may be novel therapeutic targets for inducing MSC-derived chondrogenic mass without hypertrophic phenotype.

### 6. confirm the effect of LINC02035 and LOC100130207 (3) - perform in vivo experiments

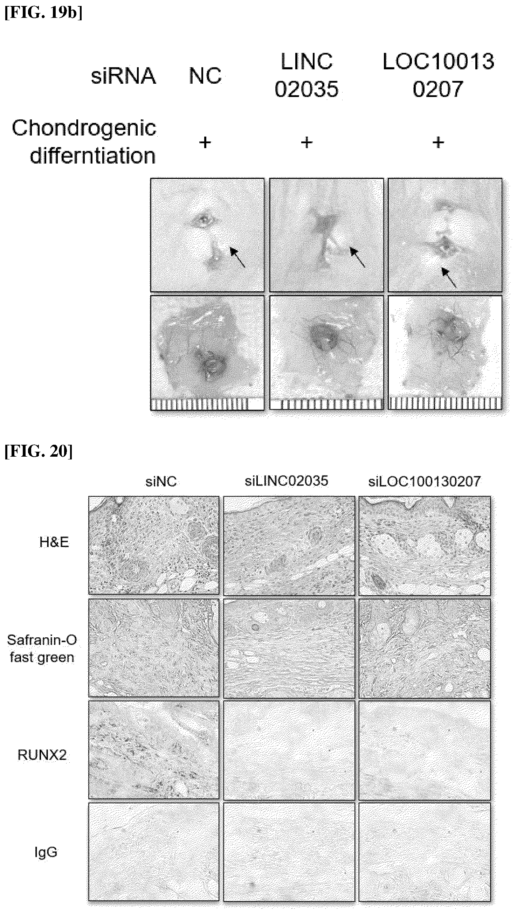

To confirm the prevention of chondrocyte hypertrophy by inhibition of the two lncRNAs in vivo, the siRNAs were transfected into human mesenchymal stem cells (hMSCs), and chondrogenic differentiation was induced for 14 days in three 3D masses (3 x 10⁵ cells/1 mass) for the siNC control group, siLINC02035 group, and siLOC100130207 group, respectively (see FIG. 19a and FIG. 19b). These were transplanted into anesthetized nude mice, and the resulting tissues were isolated for histological analysis after 7 days and subjected to safranin-O and H&E staining. Immunohistochemical staining revealed that the expression of RUNX2, a chondrocyte hypertrophy marker, was significantly reduced in the two lncRNA-silenced groups (siLNC02035 and siLOC100130207) compared to RUNX2 expression in the control siNC group (see FIG. 20). This confirms the effectiveness of silncRNAs siLNC02035 and siLOC100130207 in inhibiting chondrocyte hypertrophy in vivo, and may be useful for the treatment of arthritis by inhibiting hypertrophy.

Taken together, in addition to the degeneration and inflammatory response of articular cartilage, hypertrophic changes of chondrocytes present in articular cartilage are also one of the hallmarks of osteoarthritis, and the compositions of the present invention can prevent or delay changes such as chondrocyte hypertrophy, thereby preventing further osteoarthritis progression, and further treat osteoarthritis disease. In particular, the novel lncRNAs LINC02035 and LOC100130207 contribute significantly to RUNX2 protein stabilization, and thus are expected to be novel therapeutic targets for osteoarthritis that can be used beyond the diagnosis of osteoarthritis to slow or prevent the progression of osteoarthritis by modulating RUNX2-mediated chondrocyte hypertrophy.

Although the present invention has been described in detail with reference to the specific features, it will be apparent to those skilled in the art that this detailed description is only of a preferred embodiment thereof, and does not limit the scope of the present invention. Thus, the substantial scope of the present invention will be defined by the appended claims and equivalents thereto.

### Industrial Applicability

The compositions according to the present invention can be used to diagnose bone-related diseases by measuring the expression of novel lncRNAs, and can further be used to effectively inhibit vascularization or calcification of articular cartilage by inducing inhibition of hypertrophy of chondrocytes through inhibition of expression of the discovered novel lncRNAs, and thus can be used very effectively for prevention or treatment of bone-related diseases.

## Claims

1. A composition for diagnosing a bone-related disease, comprising an agent for measuring an expression level of long non-coding RNAs (lncRNA).

2. The composition according to claim 1, wherein the lncRNA is at least one selected from the group consisting of LINC02035, LOC100130207, LINC02593, LOC100287808, LMNTD2-AS1, HCG4B, and LOC100287175.

3. The composition according to claim 2, wherein the lncRNA is LINC02035 or LOC100130207.

4. The composition according to claim 1, wherein the agent for measuring the expression level of the lncRNA comprises at least one selected from the group consisting of a primer, a probe, and an antisense nucleotide, which bind specifically to the gene.

5. The composition according to claim 1, wherein the bone-related disease is at least one selected from the group consisting of osteoarthritis, rheumatoid arthritis, osteoporosis, osteoporosis, osteomalacia, osteopenia, osteopetrosis, bone atrophy, fibrous dysplasia, and Paget's disease, hypercalcemia, giant cell tumor, neoplastic destruction of bone, cancer-related bone resorption disease, fracture, osteolysis, and chondrocalcinosis.

6. The composition according to claim 1, wherein the bone-related disease is caused by a dysregulation of hypertrophic change in chondrocyte.

7. A kit for diagnosing a bone-related disease, comprising the composition of any one of claims 1 to 6.

8. A method for providing information for diagnosing a bone-related disease, comprising measuring an expression level of long non-coding RNAs (lncRNA) in a biological sample isolated from a subject of interest.

9. The method according to claim 8, wherein it is predicted that, when the measured expression level of long non-coding RNAs (lncRNA) is higher than a normal control, the subject of interest has a bone-related disease caused by dysregulated hypertrophy of chondrocytes.

10. The method according to claim 8, wherein the lncRNA is at least one selected from the group consisting of LINC02035, LOC100130207, LINC02593, LOC100287808, LMNTD2-AS1, HCG4B, and LOC100287175.

11. The method according to claim 8, wherein the bone-related disease is at least one selected from the group consisting of osteoarthritis, rheumatoid arthritis, osteoporosis, osteoporosis, osteomalacia, osteopenia, osteopetrosis, bone atrophy, fibrous dysplasia, and Paget's disease, hypercalcemia, giant cell tumor, neoplastic destruction of bone, cancer-related bone resorption disease, fracture, osteolysis, and chondrocalcinosis.

12. A diagnostic device for a bone-related disease comprising:
(a) a measurement unit for measuring expression levels of long non-coding RNAs (lncRNA) in a biological sample obtained from a subject of interest; and
(b) a detection unit for output from the expression level of the lncRNA measured in the measurement unit, whether or not the subject has developed or is likely to develop a bone-related disease.

13. A composition for inhibiting chondrocyte hypertrophy, comprising an agent for reducing the expression level of long non-coding RNAs (lncRNA).

14. The composition according to claim 13, wherein the lncRNA is at least one selected from the group consisting of LINC02035, LOC100130207, LINC02593, LOC100287808, LMNTD2-AS1, HCG4B, and LOC100287175.

15. The composition according to claim 13, wherein the agent for reducing the expression level lncRNA is any one or more selected from the group consisting of an antisense nucleotide, a short interfering RNA (siRNA), a short hairpin RNA, and ribozyme, which bind complementarily to the lncRNA.

16. The composition according to claim 13, wherein the composition modulates the early inflammatory response by inhibiting vascularization or calcification of articular cartilage.

17. A pharmaceutical composition for treating a bone-related disease, comprising an agent for reducing the expression level of long non-coding RNAs (lncRNA).

18. The composition according to claim 17, wherein the lncRNA is at least one selected from the group consisting of LINC02035, LOC100130207, LINC02593, LOC100287808, LMNTD2-AS1, HCG4B, and LOC100287175.

19. The composition according to claim 17, wherein the bone-related disease is at least one selected from the group consisting of osteoarthritis, rheumatoid arthritis, osteoporosis, osteoporosis, osteomalacia, osteopenia, osteopetrosis, bone atrophy, fibrous dysplasia, and Paget's disease, hypercalcemia, giant cell tumor, neoplastic destruction of bone, cancer-related bone resorption disease, fracture, osteolysis, and chondrocalcinosis.

20. The composition according to claim 17, wherein the bone-related disease is caused by a dysregulation of hypertrophic change in chondrocyte.

21. A method for screening a pharmaceutical composition for a bone-related disease comprising:
bringing a candidate substance into contact with a biological sample isolated from a subject of interest; and
measuring expression levels of at least one long non-coding RNAs (lncRNA) selected from the group consisting of LINC02035, LOC100130207, LINC02593, LOC100287808, LMNTD2-AS1, HCG4B, and LOC100287175 in the biological sample,
wherein the bone-related disease is caused by a dysregulation of hypertrophic change in chondrocyte.

22. The method according to claim 21, wherein the method of screening comprises further determining as the composition for treating the bone-related disease if the expression level of the lncRNA measured after treating with the candidate substance decreased than those of the lncRNA measured before treating with that.
